# EUROPEAN PATENT SPECIFICATION

(11) **EP 4 268 232 B1**
(45) Date of publication and mention of the grant of the patent: **25.03.2026**
(21) Application number: 21912193.6
(22) Date of filing: 22.12.2021
(51) Int. Cl.: G16B 40/20, G16B 30/00, C12Q 1/6886, C12Q 1/689, C12Q 1/70

(54) **TAXONOMY-INDEPENDENT CANCER DIAGNOSTICS AND CLASSIFICATION USING MICROBIAL NUCLEIC ACIDS AND SOMATIC MUTATIONS**
TAXONOMIEUNABHÄNGIGE KREBSDIAGNOSE UND -KLASSIFIZIERUNG MIT MIKROBIELLEN NUKLEINSÄUREN UND SOMATISCHEN MUTATIONEN
DIAGNOSTICS ET CLASSIFICATION DU CANCER INDÉPENDANTS DE LA TAXINOMIE ET FAISANT APPEL À DES ACIDES NUCLÉIQUES MICROBIENS ET À DES MUTATIONS SOMATIQUES

(30) Priority: 22.12.2020 US 202063128971 P
(43) Date of publication of application: 01.11.2023
(73) Proprietor: Universal Diagnostics, S.A., 41013 Sevilla (ES)
(72) Inventor: WANDRO, Stephen, San Diego, California 92121 (US); ADAMS, Eddie, San Diego, California 92121 (US); MILLER-MONTGOMERY, Sandrine, San Diego, California 92121 (US)
(74) Representative: Wichmann, Hendrik
(86) International application number: PCT/US2021/064977
(87) International publication number: WO 2022/140616

(56) References cited:
- WO-A1-2019/209954
- WO-A1-2020/232033
- US-A1- 2014 272 953
- US-A1- 2017 342 500
- US-A1- 2019 316 209
- US-A1- 2020 202 980
- POORE GREGORY D.; KOPYLOVA EVGUENIA; ZHU QIYUN; CARPENTER CAROLINA; FRARACCIO SERENA; WANDRO STEPHEN; KOSCIOLEK TOMASZ; JANSSEN ST: "Microbiome analyses of blood and tissues suggest cancer diagnostic approach", NATURE, NATURE PUBLISHING GROUP UK, LONDON, vol. 579, no. 7800, 1 March 2020 (2020-03-01), London, pages 567 - 574, XP037075354, ISSN: 0028-0836, DOI: 10.1038/s41586-020-2095-1

## Description

### CROSS-REFERENCE

This application claims benefit of U.S. Provisional Patent Application No. 63/128,971 filed December 22, 2020.

### BACKGROUND

An ideal diagnostic test for the detection of cancer in a subject would have the following characteristics: (i) it should identify, with high confidence, the tissue/body site location(s) of the cancer; (ii) it should identify the presence of somatic mutations that account for or are tightly associated with the cancerous state; (iii) it should detect the occurrence of cancer early (e.g., Stages I - II) to enable early-stage medical intervention; (iv) it should be minimally invasive; and (vi) it should be both highly sensitive and specific with respect to the cancer being diagnosed (i.e., there should be a high probability that the test will be positive when the cancer is present and a high probability that the test will be negative when the cancer is not present). Today, liquid biopsy-based diagnostics-both commercialized and in development-fall into two broad, nonoverlapping categories - those that can detect cancer-associated somatic mutations and those that can detect the tissue/body site location of a cancer on the basis of tissue-unique molecular patterns, such as DNA methylation. Neither category of existing diagnostics therefore provides the full complement of data that would otherwise tell a physician where to focus medical intervention and which medicaments should be selected.

Thus, there remains a need in the art for early-stage cancer diagnostics that can detect the tissue/body site location(s) of cancer with high analytic sensitivity and specificity while also determining somatic mutations associated with the detected cancer. WO 2019/209954 A1 describes methods for screening for a cancer condition of a subject by sequencing cell-free nucleic acid from the subject and determining, for each pathogen in a set of pathogens, a corresponding amount of the plurality of sequence reads that map to a sequence in a pathogen target reference for the respective pathogen, and using the set of amounts of sequence reads to determine the cancer condition. WO 2020/232033 A1 describes systems and methods for identifying a diagnosis of a cancer condition for a somatic tumor specimen of a subject.

### SUMMARY

The present invention is defined in the claims.

The present disclosure provides a method to accurately diagnose cancer, its location, and predict a cancer's likelihood of responding to certain therapies, using nucleic acids of non-human origin from a human tissue or liquid biopsy sample in combination with identified human somatic mutations present in the sample. Specifically, the present invention provides methods for identifying the presence and abundance of cancer-associated nucleic acid sequence mutations in the human genome, the presence, and abundance of non-human nucleic acid sequences that are, by virtue of their presence and abundance, characteristic of a particular cancer and the use of machine learning to first identify disease characteristic associations among the nucleic acid sequence inputs and then diagnose the disease state of a patient on the basis of these identified disease characteristic associations.

The methods of the present invention disclosed herein generate a diagnostic model capable of diagnosing and classifying the tissue/body site of origin of a cancer whilst also providing information pertaining to somatic mutations present in the cancer. In some embodiments, detection of certain somatic mutations can be highly consequential for the therapeutic treatment of said cancer. For example, recent results from a double-blind 3-year phase 3 trial demonstrated that in patients with epidermal growth factor receptor (*EGFR*) mutation positive non-small cell lung carcinoma, disease-free survival was significantly extended by treatment with an *EGFR* tyrosine kinase inhibitor (Osimertinib; PMID: 32955177). While *EGFR* oncogenic mutations are not restricted to lung cancers (being present in breast cancer and glioblastoma as well), the methods disclosed herein would not be limited to only detecting the presence of *EGFR* mutations but also, by detecting microbial nucleic acid signatures characteristic of lung cancer, would report which tissue likely harbored the cells bearing these *EGFR* mutations, thus focusing a physician's field of inquiry.

Aspects disclosed herein provide a method of creating a diagnostic cancer model comprising: (a) sequencing nucleic acid compositions of a biological sample to generate sequencing reads; (b) isolating sequencing reads to isolate a plurality of filtered sequencing reads; (c) generating a plurality of k-mers from the plurality of filtered sequencing reads; (d) determining a taxonomy independent abundance of the k-mers; (e) creating the diagnostic model by training a machine learning algorithm with the taxonomy independent abundance of the k-mers. In some embodiments, isolating is performed by exact matching between the sequencing reads and a human reference genome database. In some embodiments, exact matching comprises computationally filtering of sequencing reds with the software program Kraken or Kraken 2. In some embodiments, exact matching comprises computationally filtering of the sequencing reads with the software program bowtie 2 or any equivalent thereof. In some embodiments, the method of creating a diagnostic cancer model further comprises performing in-silico decontamination of the plurality of the filtered sequencing reads to produce a plurality of decontaminated non-human, human or any combination thereof sequencing reads. In some embodiments, determining a taxonomy independent abundance of the k-mers is performed by Jellyfish, UCLUST, GenomeTools (Tallymer), KMC2, Gerbil, DSK or any combination thereof. In some embodiments, the method of creating a diagnostic cancer model further comprises mapping human sequences of the plurality of decontaminated human sequencing reads to a build of a human reference genome database to produce a plurality of sequencing alignments. In some embodiments, mapping is performed by bowtie 2 sequence alignment tool or any equivalent thereof. In some embodiments, mapping comprises end-to-end alignment, local alignment, or any combination thereof. In some embodiments, the method of creating a diagnostic cancer model further comprises identifying cancer mutations in the plurality of sequence alignments by querying a cancer mutation database. In some embodiments, the method of creating a diagnostic cancer model further comprises generating a cancer mutation abundance table for the cancer mutations. In some embodiments, the taxonomy independent abundance of the k-mers may comprise non-human k-mers, cancer mutation abundance tables or any combination thereof. In some embodiments, the biological sample comprises a tissue, a liquid biopsy sample or any combination thereof. In some embodiments, the subject is human or a non-human mammal. In some embodiments, the nucleic acid composition comprises a total population of DNA, RNA, cell-free DNA, cell-free RNA, exosomal DNA, exosomal RNA, circulating tumor cell DNA, circulating tumor cell RNA, or any combination thereof. In some embodiments, the human reference genome database is GRCh38. In some embodiments, an output of the machine learning algorithm provides a diagnosis of a presence or an absence of cancer, a cancer body site location, cancer somatic mutations or any combination thereof associated with the presence or the absence of cancer. In some embodiments, the output of the trained machine learning algorithm comprises an analysis of the cancer mutation and k-mer abundance tables. In some embodiments, the trained machine learning algorithm is trained with a set of cancer mutation and k-mer abundances that are known to be present or absent with a characteristic abundance in a cancer of interest.

In some embodiments, the diagnostic model comprises non-human k-mer abundance of one or more of the following domains of life: bacterial, archaeal, fungal, and/or viral. In some embodiments, the diagnostic model diagnoses a category, tissue-specific location of cancer or any combination thereof. In some embodiments, the diagnostic model diagnoses one or more mutations present in the cancer. In some embodiments, the diagnostic model is configured to diagnose one or more types of cancer in the subject. In some embodiments, the diagnostic model is configured to diagnose the one or more types of cancer at a low-stage (stage I or stage II) tumor. In some embodiments, the diagnostic model is configured to diagnose one or more subtypes of cancer in the subject. In some embodiments, the diagnostic model is used to predict a stage of cancer in the subject, predict cancer prognosis in the subject or any combination thereof. In some embodiments, the diagnostic model is configured to predict a therapeutic response of the subject. In some embodiments, the diagnostic model is configured to select an optimal therapy for a particular subject. In some embodiments, the diagnostic model is configured to longitudinally model a course of one or more cancers' response to a therapy and to then adjust a treatment regimen. In some embodiments, the diagnostic model diagnoses: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma or any combination thereof. In some embodiments, the diagnostic model identifies and removes non-human noise contaminant features, while selectively retaining other non-human signal features. In some embodiments, the biological sample comprises a liquid biopsy comprising: plasma, serum, whole blood, urine, cerebral spinal fluid, saliva, sweat, tears, exhaled breath condensate or any combination thereof. In some embodiments, the cancer mutation database is derived from the Catalogue of Somatic Mutations in Cancer (COSMIC), the Cancer Genome Project (CGP), The Cancer Genome Atlas (TGCA), the International Cancer Genome Consortium (ICGC) or any combination thereof.

Aspects disclosed herein provide a method of diagnosing cancer in a subject comprising: (a) detecting a plurality of somatic mutations in a sample from a the subject; (b) detecting a plurality of non-human k-mer sequences in the sample from the subject; (c) comparing the somatic mutations and the non-human k-mer sequences of (a) and (b) with an abundance of somatic mutations and non-human k-mer sequences for a particular cancer; and (d) diagnosing cancer by providing a probability of a diagnosis of the particular cancer. In some embodiments, detecting somatic mutations further comprises counting the somatic mutations in the sample from the subject. In some embodiments, detecting non-human k-mer sequences comprises counting the non-human k-mer sequences in the sample from the subject. In some embodiments, the diagnosis is a category or location of cancer. In some embodiments, the diagnosis is one or more types of cancer in the subject. In some embodiments, the diagnosis is one or more subtypes of cancer in the subject. In some embodiments, the diagnosis is the stage of cancer in a subject and/or cancer prognosis in the subject. In some embodiments, the diagnosis is a type of cancer at low-stage (Stage I or Stage II) tumor. In some embodiments, the diagnosis is the mutation status of one or more cancers in the subject. In some embodiments, the cancer comprises: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma or any combination thereof. In some embodiments, the subject is a non-human mammal. In some embodiments, the subject is a human. In some embodiments, the subject is mammalian. In some embodiments, the k-mer presence or abundance is obtained from the following non-mammalian domains of life: viral, bacterial, archaeal, fungal or any combination thereof.

The disclosure provided herein describes a method of diagnosing cancer of a subject. The method comprises: (a) determining a plurality of somatic mutations and non-human k-mer sequences of a subject's sample; (b) comparing the plurality of somatic mutations and the plurality of non-human k-mer sequences of the subject with a plurality of somatic mutations and non-human k-mer sequences for a given cancer; and (c) diagnosing cancer of the subject by providing a probability of the presence or lack thereof cancer based at least in part on the comparison of the subject's plurality of somatic mutations and non-human k-mer sequences for the given cancer. In some embodiments, determining the plurality of somatic mutation further comprises counting somatic mutations of the subject's sample. In some embodiments, determining the plurality of non-human k-mer sequences comprises counting the non-human k-mer sequences of the subject's sample. In some embodiments, diagnosing the cancer of the subject further comprises determining a category or location of the cancer. In some embodiments, diagnosing the cancer of the subject further comprises determining one or more types of the subject's cancer. In some embodiments, diagnosing the cancer of the subject further comprises determining one or more subtypes of the subject's cancer. In some embodiments, diagnosing the cancer of the subject further comprises determining the stage of the subject's cancer, cancer prognosis, or any combination thereof. In some embodiments, diagnosing the cancer of the subject further comprises determining a type of cancer at a low-stage. In some embodiments, the type of cancer at low stage comprises stage I, or stage II cancers. In some embodiments, diagnosing the cancer of the subject further comprises determining the mutation status of the subject's cancer. In some embodiments, diagnosing the cancer of the subject further comprises determining the subject's response to therapy to treat the subject's cancer. In some embodiments, the cancer comprises: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof. In some embodiments, the subject is a non-human mammal. In some embodiments, the subject is a human. In some embodiments, the subject is a mammal. In some embodiments, the plurality of non-human k-mer sequences originate from the following non-mammalian domains of life: viral, bacterial, archaeal, fungal, or any combination thereof.

The disclosure provided herein also describes a method of diagnosing cancer of a subject using a trained predictive model. The method may comprise: (a) receiving a plurality of somatic mutations and non-human k-mer nucleic acid sequences of a first one or more subjects' nucleic acid samples; (b) providing as an input to a trained predictive model the first subjects' plurality of somatic mutations and non-human k-mer nucleic acid sequences, wherein the trained predictive model is trained with a second one or more subjects' plurality of somatic mutation nucleic acid sequences, non-human k-mer nucleic acid sequences, and corresponding clinical classifications of the second one or more subjects', and wherein the first one or more subjects and the second one or more subjects are different subjects; and (c) diagnosing cancer of the first one or more subjects based at least in part on an output of the rained predictive model. Receiving the plurality of somatic mutation nucleic acid sequences may further comprise counting somatic mutation nucleic acid sequences of the first one or more subjects' nucleic acid samples. Receiving the plurality of non-human k-mer nucleic acid sequences may further comprise counting the non-human k-mer nucleic acid sequences of the first one or more subjects' nucleic acid samples. Diagnosing the cancer of the first one or more subjects may further comprise determining a category or location of the first one or more subjects' cancers. Diagnosing the cancer of the first one or more subjects may further comprise determining one or more types of the first one or more subjects' cancer. Diagnosing the cancer of the first one or more subjects may further comprise determining one or more subtypes of the first one or more subjects' cancers. Diagnosing the cancer of the first one or more subjects may further comprise determining the first one or more subjects' stage of cancer, cancer prognosis, or any combination thereof. Diagnosing the cancer of the first one or more subjects may further comprise determining a type of cancer at a low-stage. In some embodiments, the type of cancer at low stage comprises stage I, or stage II cancers. Diagnosing the cancer of the first one or more subjects may further comprise determining the mutation status of the first one or more subjects' cancers. Diagnosing the cancer of the first one or more subjects may further comprise determining the first one or more subjects' response to therapy to treat the first one or more subjects' cancers. The cancer may comprise: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof. The first one or more subjects and second one or more subjects may be non-human mammal. The first one or more subjects and second one or more subjects may be human. The first one or more subjects may be mammal. The plurality of non-human k-mer sequences may originate from the following non-mammalian domains of life: viral, bacterial, archaeal, fungal, or any combination thereof.

The disclosure provided herein also describes a method of generating predictive cancer model. The method may comprise: (a) providing one or more nucleic acid sequencing reads of one or more subjects' biological samples; (b) filtering the one or more nucleic acid sequencing reads with a human genome database thereby producing one or more filtered sequencing reads; (c) generating a plurality of k-mers from the one or more filtered sequencing reads; and (d) generating a predictive cancer model by training a predictive model with the plurality of k-mers and corresponding clinical classification of the one or more subjects. The trained predictive model may comprise a set of cancer associated k-mers. The trained predictive model may comprise a set of non-cancer associated k-mers. The method may further comprise determining an abundance of the plurality of k-mers and training the predictive model with the abundance of the plurality of k-mers. Filtering may be performed by exact matching between the one or more nucleic acid sequencing reads and the human reference genome database. Exact matching may comprise computationally filtering of the one or more nucleic acid sequencing reads with the software program Kraken or Kraken 2; or with the software program bowtie 2 or any equivalent thereof. The method may further comprise performing in-silico decontamination of the one or more filtered sequencing reads thereby producing one or more decontaminated sequencing reads. The in-silico decontamination may identify and remove non-human contaminant features, while retaining other non-human signal features. The method may further comprise mapping the one or more decontaminated sequencing reads to a build of a human reference genome database to produce a plurality of mutated human sequence alignments. The human reference genome database may comprise GRCh38. Mapping may be performed by bowtie 2 sequence alignment tool or any equivalent thereof. Mapping may comprise end-to-end alignment, local alignment, or any combination thereof. The method may further comprise identifying cancer mutations in the plurality of mutated human sequence alignments by querying a cancer mutation database. The cancer mutation database may be derived from the Catalogue of Somatic Mutations in Cancer (COSMIC), the Cancer Genome Project (CGP), The Cancer Genome Atlas (TGCA), the International Cancer Genome Consortium (ICGC) or any combination thereof. The method may further comprise generating a cancer mutation abundance table with the cancer mutations. The plurality of k-mers may comprise non-human k-mers, human mutated k-mers, non-classified DNA k-mers, or any combination thereof. The non-human k-mers may originate from the following domains of life: bacterial, archaeal, fungal, viral, or any combination thereof. The one or more biological samples may comprise a tissue sample, a liquid biopsy sample, or any combination thereof. The liquid biopsy may comprises: plasma, serum, whole blood, urine, cerebral spinal fluid, saliva, sweat, tears, exhaled breath condensate, or any combination thereof. The one or more subjects may be human or non-human mammal. The one or more nucleic acid sequencing reads may comprise DNA, RNA, cell-free DNA, cell-free RNA, exosomal DNA, exosomal RNA, circulating tumor cell DNA, circulating tumor cell RNA, or any combination thereof. The output of the predictive cancer model may provide a diagnosis of a presence or absence of cancer, a cancer body site location, cancer somatic mutations, or any combination thereof associated with the presence or absence of cancer of a subjects. The output of the predictive cancer model may comprise an analysis of the cancer somatic mutations, the abundance of the plurality of k-mers, or any combination thereof. The trained predictive model may be trained with a set of cancer mutation and k-mer abundances that are known to be present or absent with a characteristic abundance in a cancer of interest. The predictive cancer model may be configured to determine the presence or lack thereof one or more types of cancer of a subject. The one or more types of cancer may be at a low-stage. The low-stage may comprise stage I, stage II, or any combination thereof stages of cancer. The predictive cancer model may be configured to determine the presence or lack thereof one or more subtypes of cancer of a subject. The predictive cancer model may be configured to predict a stage of cancer, predict cancer prognosis, or any combination thereof. The predictive cancer model may be configured to predict a therapeutic response of a subject when administered a therapeutic compound to treat the subject's cancer. The predictive cancer model may be configured to determine an optimal therapy to treat a subject's cancer. The predictive cancer model may be configured to longitudinally model a course of a subject's one or more cancers' response to a therapy, thereby producing a longitudinal model of the course of the subjects' one or more cancers' response to therapy. The predictive cancer model may be configured to determine an adjustment to the course of therapy of the subject's one or more cancers based at least in part on the longitudinal model. The predictive cancer model may be configured to determine the presence or lack thereof: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof cancer of a subject. Determining the abundance of the plurality of k-mers may be performed by Jellyfish, UCLUST, GenomeTools (Tallymer), KMC2, Gerbil, DSK, or any combination thereof. The clinical classification of the one or more subjects may comprise healthy, cancerous, non-cancerous disease, or any combination thereof. The one or more filtered sequencing reads may comprise non-human sequencing reads, non-matched non-human sequencing reads, or any combination thereof. The non-matched non-human sequencing reads may comprise sequencing reads that do not match to a non-human reference genome database.

The disclosure provided herein also describes a method of generating predictive cancer model. The method may comprise: (a) sequencing nucleic acid compositions of one or more subjects' biological samples thereby generating one or more sequencing reads; (b) filtering the one or more nucleic acid sequencing reads with a human genome database thereby producing one or more filtered sequencing reads; (c) generating a plurality of k-mers from the one or more filtered sequencing reads; and (d) generating a predictive cancer model by training a predictive model with the plurality of k-mers and corresponding clinical classification of the one or more subjects. The trained predictive model may comprise a set of cancer associated k-mers. The trained predictive model may comprise a set of non-cancer associated k-mers. The method may further comprise determining an abundance of the plurality of k-mers and training the predictive model with the abundance of the plurality of k-mers. Filtering may be performed by exact matching between the one or more sequencing reads and the human reference genome database. Exact matching may comprise computationally filtering of the one or more sequencing reads with the software program Kraken or Kraken 2; or with the software program bowtie 2 or any equivalent thereof. The method may further comprise performing in-silico decontamination of the one or more filtered sequencing reads thereby producing one or more decontaminated sequencing reads. The in-silico decontamination may identify and remove non-human contaminant features, while retaining other non-human signal features. The method may further comprise mapping the one or more decontaminated sequencing reads to a build of a human reference genome database to produce a plurality of mutated human sequence alignments. The human reference genome database may comprise GRCh38. Mapping may be performed by bowtie 2 sequence alignment tool or any equivalent thereof. Mapping may comprise end-to-end alignment, local alignment, or any combination thereof. The method may further comprise identifying cancer mutations in the plurality of mutated human sequence alignments by querying a cancer mutation database. The cancer mutation database may be derived from the Catalogue of Somatic Mutations in Cancer (COSMIC), the Cancer Genome Project (CGP), The Cancer Genome Atlas (TGCA), the International Cancer Genome Consortium (ICGC) or any combination thereof. The method may further comprise generating a cancer mutation abundance table with the cancer mutations. The plurality of k-mers may comprise non-human k-mers, human mutated k-mers, non-classified DNA k-mers, or any combination thereof. The non-human k-mers may originate from the following domains of life: bacterial, archaeal, fungal, viral, or any combination thereof. The one or more biological samples may comprise a tissue sample, a liquid biopsy sample, or any combination thereof. The liquid biopsy may comprise: plasma, serum, whole blood, urine, cerebral spinal fluid, saliva, sweat, tears, exhaled breath condensate, or any combination thereof. The one or more subjects may be human or non-human mammal. The nucleic acid composition may comprise DNA, RNA, cell-free DNA, cell-free RNA, exosomal DNA, exosomal RNA, circulating tumor cell DNA, circulating tumor cell RNA, or any combination thereof. The output of the predictive cancer model may provide a diagnosis of a presence or absence of cancer, a cancer body site location, cancer somatic mutations, or any combination thereof associated with the presence or absence of cancer of a subject. The output of the predictive cancer model may comprise an analysis of the cancer somatic mutations, the abundance of the plurality of k-mers, or any combination thereof. The trained predictive model may be trained with a set of cancer mutation and k-mer abundances that are known to be present or absent with a characteristic abundance in a cancer of interest. The predictive cancer model may be be configured to determine a presence or lack thereof one or more types of cancer of the a subject. The one or more types of cancer may be at a low-stage. The low-stage may comprise stage I, stage II, or any combination thereof stages of cancer. The predictive cancer model may be configured to determine the presence or lack thereof one or more subtypes of cancer of the subjects. The predictive cancer model may be configured to predict a subject's a stage of cancer, predict cancer prognosis, or any combination thereof. The predictive cancer model may be configured to predict a therapeutic response of a subject when administered a therapeutic compound to treat the subject's cancer. The predictive cancer model may be configured to determine an optimal therapy to treat a subject's cancer. The predictive cancer model may be configured to longitudinally model a course of a subject's one or more cancers' response to a therapy, thereby producing a longitudinal model of the course of the subjects' one or more cancers' response to therapy. The predictive cancer model may be configured to determine an adjustment to the course of therapy of the subject's one or more cancers based at least in part on the longitudinal model. The predictive cancer model may be configured to determine the presence or lack thereof: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof cancer of the subject. Determining the abundance of the plurality of k-mers may be performed by Jellyfish, UCLUST, GenomeTools (Tallymer), KMC2, Gerbil, DSK, or any combination thereof. The clinical classification of the one or more subjects may comprise healthy, cancerous, non-cancerous disease, or any combination thereof classifications. The one or more filtered sequencing reads may comprise non-human sequencing reads, non-matched non-human sequencing reads, or any combination thereof. The one or more filtered sequencing reads may comprise non-exact matches to a reference human genome, non-human sequencing reads, non-matched non-human sequencing reads, or any combination thereof. The non-matched non-human sequencing reads may comprise sequencing reads that do not match to a non-human reference genome database.

The disclosure provided herein also describes a computer-implemented method for utilizing a trained predictive model to determine the presence or lack thereof cancer of one or more subjects. The method may comprise: (a) receiving a plurality of somatic mutations and non-human k-mer sequences of a first one or more subjects' nucleic acid samples; (b) providing as an input to a trained predictive model the first one or more subjects' plurality of somatic mutations and non-human k-mer sequences, wherein the trained predictive model is trained with a second one or more subjects' plurality of somatic mutation sequences, non-human k-mer sequences, and corresponding clinical classifications of the second one or more subjects', and wherein the first one or more subjects and the second one or more subjects are different subjects; and (c) determining the presence or lack thereof cancer of the first one or more subjects based at least in part on an output of the trained predictive model.

Receiving the plurality of somatic mutations may further comprise counting somatic mutations of the first one or more subjects' nucleic acid samples. Receiving the plurality of non-human k-mer sequences may comprise counting the non-human k-mer sequences of the first one or more subjects' nucleic acid samples. Determining the presence or lack thereof cancer of the first one or more subjects may further comprise determining a category or location of the first one or more subjects' cancers. Determining the presence or lack thereof cancer of the first one or more subjects may further comprise determining one or more types of the first one or more subjects' cancers. Determining the presence or lack thereof cancer of the first one or more subjects may further comprise determining one or more subtypes of the first one or more subjects' cancers. Determining the presence or lack thereof cancer of the first one or more subjects may further comprise determining the stage of the cancer, cancer prognosis, or any combination thereof. Determining the presence or lack thereof cancer of the first one or more subjects may further comprise determining a type of cancer at a low stage. The type of cancer at the low-stage may comprise stage I, or stage II cancers. Determining the presence or lack thereof cancer of the first one or more subjects may further comprise determining the mutation status of the first one or more subjects' cancers. The mutation status may comprise malignant, benign, or carcinoma in situ. Determining the presence or lack thereof cancer of the first one or more subjects may further comprise determining the first one or more subjects' response to a therapy to treat the first one or more subjects' cancers.

The cancer determined by the method may comprise: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof.

The first one or more subjects and the second one or more subjects may be non-human mammal subjects. The first one or more subjects and the second one or more subjects mayb be human. The first one or more subjects and the second one or more subjects may be mammals. The plurality of non-human k-mer sequences may originate from the following non-mammalian domains of life: viral, bacterial, archaeal, fungal, or any combination thereof.

### BRIEF DESCRIPTION OF THE DRAWINGS

The patent or application file contains at least one drawing executed in color. Copies of this patent or patent application publication with color drawings (s) will be provided by the Office upon request and payment of the necessary fee.

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

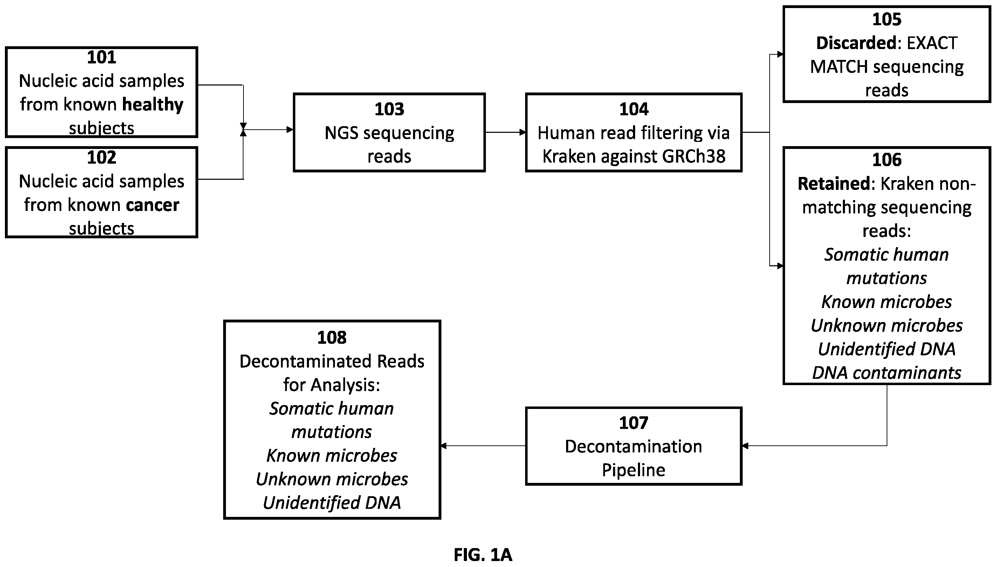

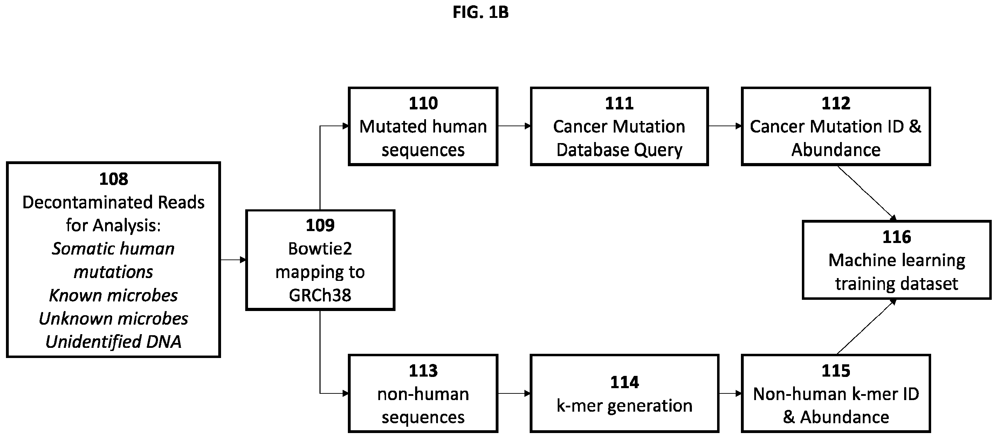

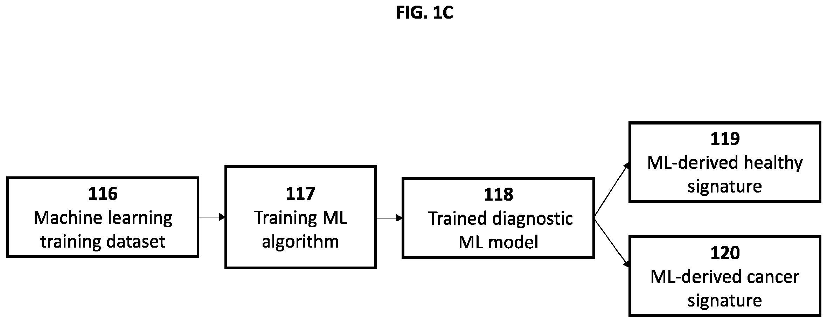

**FIGS. 1A-1C** show an example diagnostic model training scheme incorporating two analytical pipelines to enable non-human k-mer and human somatic mutation-based discovery of health and disease-associated microbial signatures. **FIG. 1A** illustrates an exemplary computational pipeline employing Kraken to prepare next generation sequencing reads for somatic mutation analysis and non-human k-mer analysis. **FIG. 1B** illustrates splitting the total pool of sequencing reads into two analytical pathways, with the resultant somatic mutation and k-mer identification and abundance tables comprising the machine learning algorithm input. **FIG. 1C** illustrates how the input from **FIG. 1B** is used to train a machine learning algorithm to generate a trained machine learning model that identifies non-human k-mer and somatic mutation signatures unique to healthy subjects and subjects with cancer.
**FIGS. 2A - 2B** show an alternative embodiment of the diagnostic model training scheme. **FIG. 2A** illustrates an exemplary computational pipeline employing Bowtie 2 to prepare next generation sequencing reads for somatic mutation analysis and non-human k-mer analysis. **FIG. 2B** illustrates splitting the total pool of sequencing reads into two analytical pathways, with the resultant somatic mutation and k-mer identification and abundance tables comprising the machine learning algorithm input.
**FIG. 3** illustrates the use of a trained model to provide a diagnosis of disease and a classification of disease state where the trained model is provided new subject data of unknown disease status.
**FIG. 4** illustrates a workflow of generating a trained cancer diagnostic model from cell free DNA sequencing reads (cfDNA) extracted k-mers comprising somatic human mutations, known microbes, unknown microbes, unidentified DNA, or any combination thereof.
**FIG. 5** shows a receiver operating characteristic curve for a predictive model trained on k-mer abundance profiles of non-mapped sequencing reads in differentiating lung cancer from lung granulomas.
**FIG. 6** shows a receiver operating characteristic curve for a predictive model trained on k-mer abundance profiles of non-mapped sequencing reads in differentiating stage one lung cancers from lung disease.
**FIG. 7** shows a computer system configured to implement training and utilizing the trained predictive models for diagnosing the presence or lack thereof cancer of a subject, as described in some embodiments herein.

### DETAILED DESCRIPTION

The disclosure provided herein describes methods and systems to diagnose and/or determine the presence or lack thereof one or more cancers of one or more subjects, the cancers subtypes, and therapy response to the one or more cancers. The diagnosis and/or determinization of the presence or lack thereof one or more cancers of one or more subjects may be completed using a combination signature of k-mer and human somatic mutation nucleic acid composition abundances. In some cases, the k-mer nucleic acid compositions may comprise non-human nucleic acid k-mers, human somatic mutation nucleic acid k-mers, non-human non-mappable k-mers (i.e., dark matter k-mers), or any combination thereof k-mers. In some instances, the diagnosis, and/or determination of the presence or lack thereof one or more cancers of one or more subjects may be accomplished by identifying specific patterns of cancer associated k-mer and/or somatic human mutations abundances of subjects with a confirmed cancer diagnosis. In some instances, one or more predictive models may be configured to determine, analyze, infer, and/or elucidate the specific patterns through training the predictive model. In some instances, the predictive model may comprise one or more machine learning models and/or algorithms. In some instances, the predictive model may comprise a cancer predictive model. In some cases, the predictive model may be trained with one or more subjects' k-mer and/or somatic human mutation abundances and the corresponding subjects' clinical classification. In some cases, the clinical classification may comprise a designation of healthy (i.e., no confirmed cancer), or cancerous (i.e., confirmed case of cancer of the subject). In some cases, the predictive model may additionally be trained with cancer specific information of the cancerous clinical classification subjects' cancer subtype, cancer body site of origin, cancer stage, prior cancer therapeutic administered and corresponding efficacy, or any combination thereof cancer specific information. In some embodiments, detected somatic human mutations that may be used for cancer classification occur within tumor suppressor genes or oncogenes, examples of which are provided in **Table 1** and **Table 2,** respectively, and their presence or abundances, in combination with k-mers, described elsewhere herein, ('a combination signature') within the sample to assign a certain probability that (1) the individual has cancer; (2) the individual has a cancer from a particular body site; (3) the individual has a particular type of cancer; and/or (4) a cancer, which may or may not be diagnosed at the time, has a high or low response to a particular cancer therapy. In some embodiments, other uses for such methods are reasonably imaginable and readily implementable to those of ordinary skill in the art.

**Table 1 Exemplary Tumor Suppressor Genes Detected and Used for Cancer Classification**

| **Hugo Symbol** | **Entrez Gene ID** | **Gene Name** | **GRCh38 RefSeq** |
|---|---|---|---|
| ABRAXAS1 | 84142 | abraxas 1, BRCA1 A complex subunit | NM_139076.2 |
| ACTG1 | 71 | actin gamma 1 | NM_001199954.1 |
| AJUBA | 84962 | ajuba LIM protein | NM_032876.5 |
| AMER1 | 139285 | APC membrane recruitment protein 1 | NM_152424.3 |
| ANKRD 11 | 29123 | ankyrin repeat domain 11 | NM_013275.5 |
| APC | 324 | APC, WNT signaling pathway regulator | NM_000038.5 |
| ARID1A | 8289 | AT-rich interaction domain 1A | NM_006015.4 |
| ARID1B | 57492 | AT-rich interaction domain 1B | NM_020732.3 |
| ARID2 | 196528 | AT-rich interaction domain 2 | NM_152641.2 |
| ARID3A | 1820 | AT-rich interaction domain 3A | NM_005224.2 |
| ARID4A | 5926 | AT-rich interaction domain 4A | NM_002892.3 |
| ARID4B | 51742 | AT-rich interaction domain 4B | NM_001206794.1 |
| ARID5B | 84159 | AT-rich interaction domain 5B | NM_032199.2 |
| ASXL1 | 171023 | additional sex combs like 1, transcriptional regulator | NM_015338.5 |
| ASXL2 | 55252 | additional sex combs like 2, transcriptional regulator | NM_018263.4 |
| ATM | 472 | ATM serine/threonine kinase | NM_000051.3 |
| ATP6V1B2 | 526 | ATPase H+ transporting V1 subunit B2 | NM_001693.3 |
| ATR | 545 | ATR serine/threonine kinase | NM_001184.3 |
| ATRX | 546 | ATRX, chromatin remodeler | NM_000489.3 |
| ATXN2 | 6311 | ataxin 2 | NM_002973.3 |
| AXIN1 | 8312 | axin 1 | NM_003502.3 |
| AXIN2 | 8313 | axin 2 | NM_004655.3 |
| B2M | 567 | beta-2-microglobulin | NM_004048.2 |
| BACH2 | 60468 | BTB domain and CNC homolog 2 | NM_001170794.1 |
| BAP1 | 8314 | BRCA1 associated protein 1 | NM_004656.3 |
| BARD1 | 580 | BRCA1 associated RING domain 1 | NM_000465.2 |
| BBC3 | 27113 | BCL2 binding component 3 | NM_001127240.2 |
| BCL10 | 8915 | B-cell CLL/lymphoma 10 | NM_003921.4 |
| BCL11B | 64919 | B-cell CLL/lymphoma 11B | NM_138576.3 |
| BCL2L11 | 10018 | BCL2 like 11 | NM_138621.4 |
| BCOR | 54880 | BCL6 corepressor | NM_001123385.1 |
| BCORL 1 | 63035 | BCL6 corepressor-like 1 | |
| BLM | 641 | Bloom syndrome RecQ like helicase | NM_000057.2 |
| BMPRIA | 657 | bone morphogenetic protein receptor type 1A | NM_004329.2 |
| BRCA1 | 672 | BRCA1, DNA repair associated | NM_007294.3 |
| BRCA2 | 675 | BRCA2, DNA repair associated | NM_000059.3 |
| BRIP1 | 83990 | BRCA1 interacting protein C-terminal helicase 1 | NM_032043.2 |
| BTG1 | 694 | BTG anti-proliferation factor 1 | NM_001731.2 |
| CASP8 | 841 | caspase 8 | NM_001080125.1 |
| CBFB | 865 | core-binding factor beta subunit | NM_022845.2 |
| CBL | 867 | Cbl proto-oncogene | NM_005188.3 |
| CCNQ | 92002 | cyclin Q | NM_152274.4 |
| CD58 | 965 | CD58 molecule | NM_001779.2 |
| CDC73 | 79577 | cell division cycle 73 | NM_024529.4 |
| CDH1 | 999 | cadherin 1 | NM_004360.3 |
| CDK12 | 51755 | cyclin dependent kinase 12 | NM_016507.2 |
| CDKN1A | 1026 | cyclin dependent kinase inhibitor 1A | NM_078467.2 |
| CDKN1B | 1027 | cyclin dependent kinase inhibitor 1B | NM_004064.3 |
| CDKN2A | 1029 | cyclin dependent kinase inhibitor 2A | NM_000077.4 |
| CDKN2B | 1030 | cyclin dependent kinase inhibitor 2B | NM_004936.3 |
| CDKN2C | 1031 | cyclin dependent kinase inhibitor 2C | NM_078626.2 |
| CEBPA | 1050 | CCAAT/enhancer binding protein alpha | NM_004364.3 |
| CHEK1 | 1111 | checkpoint kinase 1 | NM_001274.5 |
| CHEK2 | 11200 | checkpoint kinase 2 | NM_007194.3 |
| CIC | 23152 | capicua transcriptional repressor | NM_015125.3 |
| CIITA | 4261 | class II major histocompatibility complex transactivator | |
| CMTR2 | 55783 | cap methyltransferase 2 | NM_001099642.1 |
| CRBN | 51185 | cereblon | NM_016302.3 |
| CREBBP | 1387 | CREB binding protein | NM_004380.2 |
| CTCF | 10664 | CCCTC-binding factor | NM_006565.3 |
| CTR9 | 9646 | CTR9 homolog, Paf1/RNA polymerase II complex component | NM_014633.4 |
| CUL3 | 8452 | cullin 3 | NM_003590.4 |
| CUX1 | 1523 | cut like homeobox 1 | NM_181552.3 |
| CYLD | 1540 | CYLD lysine 63 deubiquitinase | NM_001042355.1 |
| DAXX | 1616 | death domain associated protein | NM_001141970.1 |
| DDX3X | 1654 | DEAD-box helicase 3, X-linked | NM_001356.4 |
| DDX41 | 51428 | DEAD-box helicase 41 | NM_016222.2 |
| DICER1 | 23405 | dicer 1, ribonuclease III | NM_030621.3 |
| DIS3 | 22894 | DIS3 homolog, exosome endoribonuclease and 3'-5' exoribonuclease | NM_014953.3 |
| DNMT3A | 1788 | DNA methyltransferase 3 alpha | NM_022552.4 |
| DNMT3B | 1789 | DNA methyltransferase 3 beta | NM_006892.3 |
| DTX1 | 1840 | deltex E3 ubiquitin ligase 1 | NM_004416.2 |
| DUSP22 | 56940 | dual specificity phosphatase 22 | NM_020185.4 |
| DUSP4 | 1846 | dual specificity phosphatase 4 | NM_001394.6 |
| ECT2L | 345930 | epithelial cell transforming 2 like | NM_001077706.2 |
| EED | 8726 | embryonic ectoderm development | NM_003797.3 |
| EGR1 | 1958 | early growth response 1 | NM_001964.2 |
| ELMSAN1 | 91748 | ELM2 and Myb/SANT domain containing 1 | NM_001043318.2 |
| EP300 | 2033 | E1A binding protein p300 | NM_001429.3 |
| EP400 | 57634 | E1A binding protein p400 | NM_015409.3 |
| EPCAM | 4072 | epithelial cell adhesion molecule | NM_002354.2 |
| EPHA3 | 2042 | EPH receptor A3 | NM_005233.5 |
| EPHB1 | 2047 | EPH receptor B1 | NM_004441.4 |
| ERCC2 | 2068 | ERCC excision repair 2, TFIIH core complex helicase subunit | NM_000400.3 |
| ERCC3 | 2071 | ERCC excision repair 3, TFIIH core complex helicase subunit | NM_000122.1 |
| ERCC4 | 2072 | ERCC excision repair 4, endonuclease catalytic subunit | NM_005236.2 |
| ERCC5 | 2073 | ERCC excision repair 5, endonuclease | NM_000123.3 |
| ERF | 2077 | ETS2 repressor factor | NM_006494.2 |
| ERRFI1 | 54206 | ERBB receptor feedback inhibitor 1 | NM_018948.3 |
| ESCO2 | 157570 | establishment of sister chromatid cohesion N-acetyltransferase 2 | NM_001017420.2 |
| ETAA1 | 54465 | Ewing tumor associated antigen 1 | NM_019002.3 |
| ETV6 | 2120 | ETS variant 6 | NM_001987.4 |
| FANCA | 2175 | Fanconi anemia complementation group A | NM_000135.2 |
| FANCC | 2176 | Fanconi anemia complementation group C | NM_000136.2 |
| FANCD2 | 2177 | Fanconi anemia complementation group D2 | NM_001018115.1 |
| FANCL | 55120 | Fanconi anemia complementation group L | NM_018062.3 |
| FAS | 355 | Fas cell surface death receptor | NM_000043.4 |
| FAT1 | 2195 | FAT atypical cadherin 1 | NM_005245.3 |
| FBXO11 | 80204 | F-box protein 11 | NM_001190274.1 |
| FBXW7 | 55294 | F-box and WD repeat domain containing 7 | NM_033632.3 |
| FH | 2271 | fumarate hydratase | NM_000143.3 |
| FLCN | 201163 | folliculin | NM_144997.5 |
| FOXO1 | 2308 | forkhead box O1 | NM_002015.3 |
| FUBP1 | 8880 | far upstream element binding protein 1 | NM_003902.3 |
| GPS2 | 2874 | G protein pathway suppressor 2 | NM_004489.4 |
| GRIN2A | 2903 | glutamate ionotropic receptor NMDA type subunit 2A | NM_001134407.1 |
| HIST1H1B | 3009 | histone cluster 1 H1 family member b | NM_005322.2 |
| HIST1H1D | 3007 | histone cluster 1 H1 family member d | NM_005320.2 |
| HLA-A | 3105 | major histocompatibility complex, class I, A | NM_001242758.1 |
| HLA-B | 3106 | major histocompatibility complex, class I, B | NM_005514.6 |
| HLA-C | 3107 | major histocompatibility complex, class I, C | NM_002117.5 |
| HNF1A | 6927 | HNF1 homeobox A | NM_000545.5 |
| ID3 | 3399 | inhibitor of DNA binding 3, HLH protein | NM_002167.4 |
| IFNGR1 | 3459 | interferon gamma receptor 1 | NM_000416.2 |
| INHA | 3623 | inhibin alpha subunit | NM_002191.3 |
| INPP4B | 8821 | inositol polyphosphate-4-phosphatase type II B | NM_001101669.1 |
| INPPL1 | 3636 | inositol polyphosphate phosphatase like 1 | NM_001567.3 |
| IRF1 | 3659 | interferon regulatory factor 1 | NM_002198.2 |
| IRF8 | 3394 | interferon regulatory factor 8 | NM_002163.2 |
| KDMS C | 8242 | lysine demethylase 5C | NM_004187.3 |
| KDM6A | 7403 | lysine demethylase 6A | NM_021140.2 |
| KEAP1 | 9817 | kelch like ECH associated protein 1 | NM_203500.1 |
| KLF2 | 10365 | Kruppel like factor 2 | NM_016270.2 |
| KLF3 | 51274 | Kruppel like factor 3 | NM_016531.5 |
| KMT2A | 4297 | lysine methyltransferase 2A | NM_001197104.1 |
| KMT2B | 9757 | lysine methyltransferase 2B | NM_014727.1 |
| KMT2C | 58508 | lysine methyltransferase 2C | NM_170606.2 |
| KMT2D | 8085 | lysine methyltransferase 2D | NM_003482.3 |
| LATS1 | 9113 | large tumor suppressor kinase 1 | NM_004690.3 |
| LATS2 | 26524 | large tumor suppressor kinase 2 | NM_014572.2 |
| LZTR1 | 8216 | leucine zipper like transcription regulator 1 | NM_006767.3 |
| MAP2K4 | 6416 | mitogen-activated protein kinase 4 | NM_003010.3 |
| MAP3K 1 | 4214 | mitogen-activated protein kinase kinase kinase 1 | NM_005921.1 |
| MAX | 4149 | MYC associated factor X | NM_002382.4 |
| MBD6 | 114785 | methyl-CpG binding domain protein 6 | NM_052897.3 |
| MEN1 | 4221 | menin 1 | NM_130799 |
| MGA | 23269 | MGA, MAX dimerization protein | NM_001164273.1 |
| MLH1 | 4292 | mutL homolog 1 | NM_000249.3 |
| MOB3B | 79817 | MOB kinase activator 3B | NM_024761.4 |
| MRE11 | 4361 | MRE11 homolog, double strand break repair nuclease | NM_005591.3 |
| MSH2 | 4436 | mutS homolog 2 | NM_000251.2 |
| MSH3 | 4437 | mutS homolog 3 | NM_002439.4 |
| MSH6 | 2956 | mutS homolog 6 | NM_000179.2 |
| MST1 | 4485 | macrophage stimulating 1 | NM_020998.3 |
| MTAP | 4507 | methylthioadenosine phosphorylase | NM_002451.3 |
| MUTYH | 4595 | mutY DNA glycosylase | NM_001048171.1 |
| NBN | 4683 | nibrin | NM_002485.4 |
| NCOR1 | 9611 | nuclear receptor corepressor 1 | NM_006311.3 |
| NF1 | 4763 | neurofibromin 1 | NM_000267 |
| NF2 | 4771 | neurofibromin 2 | NM_000268.3 |
| NFKBIA | 4792 | NFKB inhibitor alpha | NM_020529.2 |
| NKX3-1 | 4824 | NK3 homeobox 1 | NM_006167.3 |
| NPM1 | 4869 | nucleophosmin | NM_002520.6 |
| NTHL1 | 4913 | nth like DNA glycosylase 1 | NM_002528.5 |
| P2RY8 | 286530 | purinergic receptor P2Y8 | NM_178129.4 |
| PALB2 | 79728 | partner and localizer of BRCA2 | NM_024675.3 |
| PARP1 | 142 | poly | NM_001618.3 |
| PAX5 | 5079 | paired box 5 | NM_016734.2 |
| PBRM1 | 55193 | polybromo 1 | NM_018313.4 |
| PDS5B | 23047 | PDS5 cohesin associated factor B | NM_015032.3 |
| PHF6 | 84295 | PHD finger protein 6 | NM_001015877.1 |
| PHOX2B | 8929 | paired like homeobox 2b | NM_003924.3 |
| PIGA | 5277 | phosphatidylinositol glycan anchor biosynthesis class A | NM_002641.3 |
| PIK3R1 | 5295 | phosphoinositide-3-kinase regulatory subunit 1 | NM_181523.2 |
| PIK3R2 | 5296 | phosphoinositide-3-kinase regulatory subunit 2 | NM_005027.3 |
| PIK3R3 | 8503 | phosphoinositide-3-kinase regulatory subunit 3 | NM_003629.3 |
| PMAIP1 | 5366 | phorbol-12-myristate-13-acetate-induced protein 1 | NM_021127.2 |
| PMS1 | 5378 | PMS1 homolog 1, mismatch repair system component | NM_000534.4 |
| PMS2 | 5395 | PMS1 homolog 2, mismatch repair system component | NM_000535.5 |
| POLD1 | 5424 | DNA polymerase delta 1, catalytic subunit | NM_002691.3 |
| POLE | 5426 | DNA polymerase epsilon, catalytic subunit | NM_006231.2 |
| POT1 | 25913 | protection of telomeres 1 | NM_015450.2 |
| PPP2R1A | 5518 | protein phosphatase 2 scaffold subunit Aalpha | NM_014225.5 |
| PPP2R2A | 5520 | protein phosphatase 2 regulatory subunit Balpha | NM_002717.3 |
| PPP6C | 5537 | protein phosphatase 6 catalytic subunit | NM_002721.4 |
| PRDM1 | 639 | PR/SET domain 1 | NM_001198.3 |
| PRKN | 5071 | parkin RBR E3 ubiquitin protein ligase | NM_004562.2 |
| PTCH1 | 5727 | patched 1 | NM_000264.3 |
| PTEN | 5728 | phosphatase and tensin homolog | NM_000314.4 |
| PTPN2 | 5771 | protein tyrosine phosphatase, non-receptor type 2 | NM_002828.3 |
| PTPRD | 5789 | protein tyrosine phosphatase, receptor type D | NM_002839.3 |
| PTPRS | 5802 | protein tyrosine phosphatase, receptor type S | NM_002850.3 |
| PTPRT | 11122 | protein tyrosine phosphatase, receptor type T | NM_133170.3 |
| RAD21 | 5885 | RAD21 cohesin complex component | NM_006265.2 |
| RAD50 | 10111 | RAD50 double strand break repair protein | NM_005732.3 |
| RAD51 | 5888 | RAD51 recombinase | NM_002875.4 |
| RAD51B | 5890 | RAD51 paralog B | NM_133509.3 |
| RAD51C | 5889 | RAD51 paralog C | NM_058216.2 |
| RAD51D | 5892 | RAD51 paralog D | NM_002878 |
| RASA1 | 5921 | RAS p21 protein activator 1 | NM_002890.2 |
| RB1 | 5925 | RB transcriptional corepressor 1 | NM_000321.2 |
| RBM10 | 8241 | RNA binding motif protein 10 | NM_001204468.1 |
| RECQL | 5965 | RecQ like helicase | NM_032941.2 |
| RECQL4 | 9401 | RecQ like helicase 4 | ENST00000428558 |
| REST | 5978 | RE1 silencing transcription factor | NM_001193508.1 |
| RNF43 | 54894 | ring finger protein 43 | NM_017763.4 |
| ROBO1 | 6091 | roundabout guidance receptor 1 | NM_002941.3 |
| RTEL1 | 51750 | regulator of telomere elongation helicase 1 | NM_032957.4 |
| RUNX1 | 861 | runt related transcription factor 1 | NM_001754.4 |
| RYBP | 23429 | RING1 and YY1 binding protein | NM_012234.5 |
| SAMHD1 | 25939 | SAM and HD domain containing deoxynucleoside triphosphate triphosphohydrolase 1 | NM_015474.3 |
| SDHA | 6389 | succinate dehydrogenase complex flavoprotein subunit A | NM_004168.2 |
| SDHAF2 | 54949 | succinate dehydrogenase complex assembly factor 2 | NM_017841.2 |
| SDHB | 6390 | succinate dehydrogenase complex iron sulfur subunit B | NM_003000.2 |
| SDHC | 6391 | succinate dehydrogenase complex subunit C | NM_003001.3 |
| SDHD | 6392 | succinate dehydrogenase complex subunit D | NM_003002.3 |
| SESN1 | 27244 | sestrin 1 | NM_014454.2 |
| SESN2 | 83667 | sestrin 2 | NM_031459.4 |
| SESN3 | 143686 | sestrin 3 | NM_144665.3 |
| SETD2 | 29072 | SET domain containing 2 | NM_014159.6 |
| SETDB2 | 83852 | SET domain bifurcated 2 | NM_031915.2 |
| SFRP1 | 6422 | secreted frizzled related protein 1 | NM_003012.4 |
| SH2B3 | 10019 | SH2B adaptor protein 3 | NM_005475.2 |
| SH2D1A | 4068 | SH2 domain containing 1A | NM_002351.4 |
| SHQ1 | 55164 | SHQ1, H/ACA ribonucleoprotein assembly factor | NM_018130.2 |
| SLFN 11 | 91607 | schlafen family member 11 | NM_001104587.1 |
| SLX4 | 84464 | SLX4 structure-specific endonuclease subunit | NM_032444.2 |
| SMAD2 | 4087 | SMAD family member 2 | NM_001003652.3 |
| SMAD3 | 4088 | SMAD family member 3 | NM_005902.3 |
| SMAD4 | 4089 | SMAD family member 4 | NM_005359.5 |
| SMARCA2 | 6595 | SWI/SNF related, matrix associated, actin dependent regulator of chromatin, subfamily a, member 2 | NM_001289396.1 |
| SMARCA4 | 6597 | SWI/SNF related, matrix associated, actin dependent regulator of chromatin, subfamily a, member 4 | NM_001128849 |
| SMARCB1 | 6598 | SWI/SNF related, matrix associated, actin dependent regulator of chromatin, subfamily b, member 1 | NM_003073.3 |
| SMC1A | 8243 | structural maintenance of chromosomes 1A | NM_006306.3 |
| SMC3 | 9126 | structural maintenance of chromosomes 3 | NM_005445.3 |
| SMG1 | 23049 | SMG1, nonsense mediated mRNA decay associated PI3K related kinase | NM_015092.4 |
| SOCS1 | 8651 | suppressor of cytokine signaling 1 | NM_003745.1 |
| SOCS3 | 9021 | suppressor of cytokine signaling 3 | NM_003955.4 |
| SOX17 | 64321 | SRY-box 17 | NM_022454.3 |
| SP140 | 11262 | SP140 nuclear body protein | NM_007237.4 |
| SPEN | 23013 | spen family transcriptional repressor | NM_015001.2 |
| SPOP | 8405 | speckle type BTB/POZ protein | NM_001007228.1 |
| SPRED1 | 161742 | sprouty related EVH1 domain containing 1 | NM_152594.2 |
| SPRTN | 83932 | SprT-like N-terminal domain | NM_032018.6 |
| STAG1 | 10274 | stromal antigen 1 | NM_005862.2 |
| STAG2 | 10735 | stromal antigen 2 | NM_001042749.1 |
| STK11 | 6794 | serine/threonine kinase 11 | NM_000455.4 |
| SUFU | 51684 | SUFU negative regulator of hedgehog signaling | NM_016169.3 |
| SUZ12 | 23512 | SUZ12 polycomb repressive complex 2 subunit | NM_015355.2 |
| TBL1XR1 | 79718 | transducin beta like 1 X-linked receptor 1 | NM_024665.4 |
| TBX3 | 6926 | T-box 3 | NM_016569.3 |
| TCF3 | 6929 | transcription factor 3 | NM_001136139.2 |
| TCF7L2 | 6934 | transcription factor 7 like 2 | NM_001146274.1 |
| TENT5C | 54855 | terminal nucleotidyltransferase 5C | NM_017709.3 |
| TET1 | 80312 | tet methylcytosine dioxygenase 1 | NM_030625.2 |
| TET2 | 54790 | tet methylcytosine dioxygenase 2 | NM_001127208.2 |
| TET3 | 200424 | tet methylcytosine dioxygenase 3 | NM_144993 |
| TGFBR1 | 7046 | transforming growth factor beta receptor 1 | NM_004612.2 |
| TGFBR2 | 7048 | transforming growth factor beta receptor 2 | NM_003242 |
| TMEM127 | 55654 | transmembrane protein 127 | NM_001193304.2 |
| TNFAIP3 | 7128 | TNF alpha induced protein 3 | NM_006290.3 |
| TNFRSF14 | 8764 | TNF receptor superfamily member 14 | NM_003820.2 |
| TOP1 | 7150 | topoisomerase | NM_003286.2 |
| TP53 | 7157 | tumor protein p53 | NM_000546.5 |
| TP53BP1 | 7158 | tumor protein p53 binding protein 1 | NM_001141980.1 |
| TRAF 3 | 7187 | TNF receptor associated factor 3 | NM_003300.3 |
| TRAF5 | 7188 | TNF receptor associated factor 5 | NM_001033910.2 |
| TSC1 | 7248 | tuberous sclerosis 1 | NM_000368.4 |
| TSC2 | 7249 | tuberous sclerosis 2 | NM_000548.3 |
| VHL | 7428 | von Hippel-Lindau tumor suppressor | NM_000551.3 |
| WIF1 | 11197 | WNT inhibitory factor 1 | NM_007191.4 |
| XRCC2 | 7516 | X-ray repair cross complementing 2 | NM_005431.1 |
| ZFHX3 | 463 | zinc finger homeobox 3 | NM_006885.3 |
| ZFP36L1 | 677 | ZFP36 ring finger protein like 1 | NM_001244698.1 |
| ZNF750 | 79755 | zinc finger protein 750 | NM_024702.2 |
| ZNRF3 | 84133 | zinc and ring finger 3 | NM_001206998.1 |

**Table 2 Exemplary Oncogenes Detected and Used for Cancer Classification**

| **Hugo Symbol** | **Entrez Gene ID** | **Gene Name** | **GRCh38 RefSeq** |
|---|---|---|---|
| ABL1 | 25 | ABL proto-oncogene 1, non-receptor tyrosine kinase | NM_005157.4 |
| ABL2 | 27 | ABL proto-oncogene 2, non-receptor tyrosine kinase | NM_007314.3 |
| ACVR1 | 90 | activin A receptor type 1 | NM_001111067.2 |
| AGO1 | 26523 | argonaute 1, RISC catalytic component | NM_012199.2 |
| AKT1 | 207 | AKT serine/threonine kinase 1 | NM_001014431.1 |
| AKT2 | 208 | AKT serine/threonine kinase 2 | NM_001626.4 |
| AKT3 | 10000 | AKT serine/threonine kinase 3 | NM_005465.4 |
| ALK | 238 | anaplastic lymphoma receptor tyrosine kinase | NM_004304.4 |
| ALOX12B | 242 | arachidonate 12-lipoxygenase, 12R type | NM_001139.2 |
| APLNR | 187 | apelin receptor | NM_005161.4 |
| AR | 367 | androgen receptor | NM_000044.3 |
| ARAF | 369 | A-Raf proto-oncogene, serine/threonine kinase | NM_001654.4 |
| ARHGAP35 | 2909 | Rho GTPase activating protein 35 | NM_004491.4 |
| ARHGEF28 | 64283 | Rho guanine nucleotide exchange factor 28 | NM_001177693.1 |
| ARID3B | 10620 | AT-rich interaction domain 3B | NM_001307939.1 |
| ATF1 | 466 | activating transcription factor 1 | NM_005171.4 |
| ATXN7 | 6314 | ataxin 7 | NM_000333.3 |
| AURKA | 6790 | aurora kinase A | NM_003600.2 |
| AURKB | 9212 | aurora kinase B | NM_004217.3 |
| AXL | 558 | AXL receptor tyrosine kinase | NM_021913.4 |
| BCL2 | 596 | BCL2, apoptosis regulator | NM_000633.2 |
| BCL6 | 604 | B-cell CLL/lymphoma 6 | NM_001706.4 |
| BCL9 | 607 | B-cell CLL/lymphoma 9 | NM_004326.3 |
| BCR | 613 | BCR, RhoGEF and GTPase activating protein | NM_004327.3 |
| BRAF | 673 | B-Raf proto-oncogene, serine/threonine kinase | NM_004333.4 |
| BRD4 | 23476 | bromodomain containing 4 | NM_058243.2 |
| BTK | 695 | Bruton tyrosine kinase | NM_00061.2 |
| CALR | 811 | calreticulin | NM_004343.3 |
| CARD 11 | 84433 | caspase recruitment domain family member 11 | NM_032415.4 |
| CCNB3 | 85417 | cyclin B3 | NM_033031.2 |
| CCND1 | 595 | cyclin D1 | NM_053056.2 |
| CCND2 | 894 | cyclin D2 | NM_001759.3 |
| CCND3 | 896 | cyclin D3 | NM_001760.3 |
| CCNE1 | 898 | cyclin E1 | NM_001238.2 |
| CD274 | 29126 | CD274 molecule | NM_014143.3 |
| CD276 | 80381 | CD276 molecule | NM_001024736.1 |
| CD28 | 940 | CD28 molecule | NM_006139.3 |
| CD79A | 973 | CD79a molecule | NM_001783.3 |
| CD79B | 974 | CD79b molecule | NM_001039933.1 |
| CDC42 | 998 | cell division cycle 42 | NM_001791.3 |
| CDK4 | 1019 | cyclin dependent kinase 4 | NM_000075.3 |
| CDK6 | 1021 | cyclin dependent kinase 6 | NM_001145306.1 |
| CDK8 | 1024 | cyclin dependent kinase 8 | NM_001260.1 |
| COP1 | 64326 | COP1 E3 ubiquitin ligase | NM_022457.5 |
| CREB1 | 1385 | cAMP responsive element binding protein 1 | NM_134442.3 |
| CRKL | 1399 | CRK like proto-oncogene, adaptor protein | NM_005207.3 |
| CRLF2 | 64109 | cytokine receptor-like factor 2 | NM_022148.2 |
| CSF3R | 1441 | colony stimulating factor 3 receptor | NM_000760.3 |
| CTLA4 | 1493 | cytotoxic T-lymphocyte associated protein 4 | NM_005214.4 |
| CTNNB1 | 1499 | catenin beta 1 | NM_001904.3 |
| CXCR4 | 7852 | C-X-C motif chemokine receptor 4 | NM_003467.2 |
| CXORF67 | 340602 | chromosome X open reading frame 67 | NM_203407.2 |
| CYP19A1 | 1588 | cytochrome P450 family 19 subfamily A member 1 | NM_000103.3 |
| CYSLTR2 | 57105 | cysteinyl leukotriene receptor 2 | NM_020377.2 |
| DCUN1D1 | 54165 | defective in cullin neddylation 1 domain containing 1 | NM_020640.2 |
| DDR2 | 4921 | discoidin domain receptor tyrosine kinase 2 | NM_006182.2 |
| DDX4 | 54514 | DEAD-box helicase 4 | NM_024415.2 |
| DEK | 7913 | DEK proto-oncogene | NM_003472.3 |
| DNMT1 | 1786 | DNA methyltransferase 1 | NM_001379.2 |
| DOT1L | 84444 | DOT1 like histone lysine methyltransferase | NM_032482.2 |
| E2F3 | 1871 | E2F transcription factor 3 | NM_001949.4 |
| EGFL7 | 51162 | EGF like domain multiple 7 | NM_201446.2 |
| EGFR | 1956 | epidermal growth factor receptor | NM_005228.3 |
| EIF4A2 | 1974 | eukaryotic translation initiation factor 4A2 | NM_001967.3 |
| EIF4E | 1977 | eukaryotic translation initiation factor 4E | NM_001130678.1 |
| ELF3 | 1999 | E74 like ETS transcription factor 3 | NM_004433.4 |
| EPHA7 | 2045 | EPH receptor A7 | NM_004440.3 |
| EPOR | 2057 | erythropoietin receptor | NM_000121.3 |
| ERBB2 | 2064 | erb-b2 receptor tyrosine kinase 2 | NM_004448.2 |
| ERBB3 | 2065 | erb-b2 receptor tyrosine kinase 3 | NM_001982.3 |
| ERBB4 | 2066 | erb-b2 receptor tyrosine kinase 4 | NM_005235.2 |
| ERG | 2078 | ERG, ETS transcription factor | NM_182918.3 |
| ESR1 | 2099 | estrogen receptor 1 | NM_001122740.1 |
| ETV1 | 2115 | ETS variant 1 | NM_001163147.1 |
| ETV4 | 2118 | ETS variant 4 | NM_001079675.2 |
| ETV5 | 2119 | ETS variant 5 | NM_004454.2 |
| EWSR1 | 2130 | EWS RNA binding protein 1 | NM_005243.3 |
| EZH1 | 2145 | enhancer of zeste 1 polycomb repressive complex 2 subunit | NM_001991.3 |
| EZH2 | 2146 | enhancer of zeste 2 polycomb repressive complex 2 subunit | NM_004456.4 |
| FGF19 | 9965 | fibroblast growth factor 19 | NM_005117.2 |
| FGF3 | 2248 | fibroblast growth factor 3 | NM_005247.2 |
| FGF4 | 2249 | fibroblast growth factor 4 | NM_002007.2 |
| FGFR1 | 2260 | fibroblast growth factor receptor 1 | NM_001174067.1 |
| FGFR2 | 2263 | fibroblast growth factor receptor 2 | NM_000141.4 |
| FGFR3 | 2261 | fibroblast growth factor receptor 3 | NM_000142.4 |
| FGFR4 | 2264 | fibroblast growth factor receptor 4 | NM_213647.1 |
| FLI1 | 2313 | Fli-1 proto-oncogene, ETS transcription factor | NM_002017.4 |
| FLT1 | 2321 | fms related tyrosine kinase 1 | NM_002019.4 |
| FLT3 | 2322 | fms related tyrosine kinase 3 | NM_004119.2 |
| FLT4 | 2324 | fms related tyrosine kinase 4 | NM_182925.4 |
| FOXA1 | 3169 | forkhead box A1 | NM_004496.3 |
| FOXF1 | 2294 | forkhead box F1 | NM_001451.2 |
| FOXL2 | 668 | forkhead box L2 | NM_023067.3 |
| FOXP1 | 27086 | forkhead box P1 | NM_001244814.1 |
| FURIN | 5045 | furin, paired basic amino acid cleaving enzyme | NM_001289823.1 |
| FYN | 2534 | FYN proto-oncogene, Src family tyrosine kinase | NM_153047.3 |
| GAB1 | 2549 | GRB2 associated binding protein 1 | NM_002039.3 |
| GAB2 | 9846 | GRB2 associated binding protein 2 | NM_080491.2 |
| GATA2 | 2624 | GATA binding protein 2 | NM_032638.4 |
| GATA3 | 2625 | GATA binding protein 3 | NM_002051.2 |
| GLI1 | 2735 | GLI family zinc finger 1 | NM_005269.2 |
| GNA11 | 2767 | G protein subunit alpha 11 | NM_002067.2 |
| GNA12 | 2768 | G protein subunit alpha 12 | NM_007353.2 |
| GNA13 | 10672 | G protein subunit alpha 13 | NM_006572.5 |
| GNAQ | 2776 | G protein subunit alpha q | NM_002072.3 |
| GNAS | 2778 | GNAS complex locus | NM_000516.4 |
| GNB1 | 2782 | G protein subunit beta 1 | NM_001282539.1 |
| GREM1 | 26585 | gremlin 1, DAN family BMP antagonist | NM_013372.6 |
| GSK3B | 2932 | glycogen synthase kinase 3 beta | NM_002093.3 |
| GTF2I | 2969 | general transcription factor IIi | NM_032999.3 |
| H3-3A | 3020 | H3.3 histone A | NM_002107.4 |
| HDAC1 | 3065 | histone deacetylase 1 | NM_004964.2 |
| HDAC4 | 9759 | histone deacetylase 4 | NM_006037.3 |
| HDAC7 | 51564 | histone deacetylase 7 | XM_011538481.1 |
| HGF | 3082 | hepatocyte growth factor | NM_000601.4 |
| HIF1A | 3091 | hypoxia inducible factor 1 alpha subunit | NM_001530.3 |
| HIST1H1E | 3008 | histone cluster 1 H1 family member e | NM_005321.2 |
| HIST1H2AM | 8336 | histone cluster 1 H2A family member m | NM_003514 |
| HOXB13 | 10481 | homeobox B13 | NM_006361.5 |
| HRAS | 3265 | HRas proto-oncogene, GTPase | NM_001130442.1 |
| ICOSLG | 23308 | inducible T-cell costimulator ligand | NM_015259.4 |
| IDH1 | 3417 | isocitrate dehydrogenase | NM_005896.2 |
| IDH2 | 3418 | isocitrate dehydrogenase | NM_002168.2 |
| IGF1 | 3479 | insulin like growth factor 1 | NM_001111285.1 |
| IGF1R | 3480 | insulin like growth factor 1 receptor | NM_000875.3 |
| IGF2 | 3481 | insulin like growth factor 2 | NM_001127598.1 |
| IKBKE | 9641 | inhibitor of kappa light polypeptide gene enhancer in B-cells, kinase epsilon | NM_014002.3 |
| IKZF3 | 22806 | IKAROS family zinc finger 3 | NM_012481.4 |
| IL3 | 3562 | interleukin 3 | NM_000588.3 |
| IL7R | 3575 | interleukin 7 receptor | NM_002185.3 |
| INHBA | 3624 | inhibin beta A subunit | NM_002192.2 |
| INSR | 3643 | insulin receptor | NM_000208.2 |
| IRF4 | 3662 | interferon regulatory factor 4 | NM_002460.3 |
| IRS 1 | 3667 | insulin receptor substrate 1 | NM_005544.2 |
| IRS2 | 8660 | insulin receptor substrate 2 | NM_003749.2 |
| JAK1 | 3716 | Janus kinase 1 | NM_002227.2 |
| JAK2 | 3717 | Janus kinase 2 | NM_004972.3 |
| JAK3 | 3718 | Janus kinase 3 | NM_000215.3 |
| JARID2 | 3720 | jumonji and AT-rich interaction domain containing 2 | NM_004973.3 |
| JUN | 3725 | Jun proto-oncogene, AP-1 transcription factor subunit | NM_002228.3 |
| KDM5A | 5927 | lysine demethylase 5A | NM_001042603.1 |
| KDR | 3791 | kinase insert domain receptor | NM_002253.2 |
| KIT | 3815 | KIT proto-oncogene receptor tyrosine kinase | NM_000222.2 |
| KLF4 | 9314 | Kruppel like factor 4 | NM_004235.4 |
| KLF5 | 688 | Kruppel like factor 5 | NM_001730.4 |
| KRAS | 3845 | KRAS proto-oncogene, GTPase | NM_004985 |
| KSR2 | 283455 | kinase suppressor of ras 2 | |
| LCK | 3932 | LCK proto-oncogene, Src family tyrosine kinase | NM_001042771.2 |
| LMO1 | 4004 | LIM domain only 1 | NM_002315.2 |
| LMO2 | 4005 | LIM domain only 2 | NM_001142315.1 |
| LRP5 | 4041 | LDL receptor related protein 5 | NM_001291902.1 |
| LRP6 | 4040 | LDL receptor related protein 6 | NM_002336.2 |
| LTB | 4050 | lymphotoxin beta | NM_002341.1 |
| LYN | 4067 | LYN proto-oncogene, Src family tyrosine kinase | NM_002350.3 |
| MAD2L2 | 10459 | MAD2 mitotic arrest deficient-like 2 | NM_001127325.1 |
| MAFB | 9935 | MAF bZIP transcription factor B | NM_005461.4 |
| MAP2K 1 | 5604 | mitogen-activated protein kinase kinase 1 | NM_002755.3 |
| MAP2K2 | 5605 | mitogen-activated protein kinase kinase 2 | NM_030662.3 |
| MAP3K13 | 9175 | mitogen-activated protein kinase kinase kinase 13 | NM_004721.4 |
| MAP3K14 | 9020 | mitogen-activated protein kinase kinase kinase 14 | NM_003954.3 |
| MAPK1 | 5594 | mitogen-activated protein kinase 1 | NM_002745.4 |
| MAPK3 | 5595 | mitogen-activated protein kinase 3 | NM_002746.2 |
| MCL1 | 4170 | BCL2 family apoptosis regulator | NM_021960.4 |
| MDM2 | 4193 | MDM2 proto-oncogene | NM_002392.5 |
| MDM4 | 4194 | MDM4, p53 regulator | NM_002393.4 |
| MECOM | 2122 | MDS1 and EVI1 complex locus | NM_001105078.3 |
| MED 12 | 9968 | mediator complex subunit 12 | NM_005120.2 |
| MEF2B | 1002718 49 | myocyte enhancer factor 2B | NM_001145785.1 |
| MEF2D | 4209 | myocyte enhancer factor 2D | NM_005920.3 |
| MET | 4233 | MET proto-oncogene, receptor tyrosine kinase | NM_000245.2 |
| MGAM | 8972 | maltase-glucoamylase | NM_004668.2 |
| MITF | 4286 | melanogenesis associated transcription factor | NM_000248 |
| MLLT10 | 8028 | myeloid/lymphoid or mixed-lineage leukemia; translocated to, 10 | NM_001195626.1 |
| MPL | 4352 | MPL proto-oncogene, thrombopoietin receptor | NM_005373.2 |
| MSI1 | 4440 | musashi RNA binding protein 1 | NM_002442.3 |
| MSI2 | 124540 | musashi RNA binding protein 2 | NM_138962.2 |
| MST1R | 4486 | macrophage stimulating 1 receptor | NM_002447.2 |
| MTOR | 2475 | mechanistic target of rapamycin | NM_004958.3 |
| MYC | 4609 | v-myc avian myelocytomatosis viral oncogene homolog | NM_002467.4 |
| MYCL | 4610 | v-myc avian myelocytomatosis viral oncogene lung carcinoma derived homolog | NM_001033082.2 |
| MYCN | 4613 | v-myc avian myelocytomatosis viral oncogene neuroblastoma derived homolog | NM_005378.4 |
| MYD88 | 4615 | myeloid differentiation primary response 88 | NM_002468.4 |
| NADK | 65220 | NAD kinase | NM_001198993.1 |
| NCOA3 | 8202 | nuclear receptor coactivator 3 | NM_181659.2 |
| NCSTN | 23385 | nicastrin | NM_015331.2 |
| NFE2 | 4778 | nuclear factor, erythroid 2 | NM_001136023.2 |
| NFE2L2 | 4780 | nuclear factor, erythroid 2 like 2 | NM_006164.4 |
| NKX2-1 | 7080 | NK2 homeobox 1 | NM_001079668.2 |
| NOTCH1 | 4851 | notch 1 | NM_017617.3 |
| NOTCH2 | 4853 | notch 2 | NM_024408.3 |
| NOTCH3 | 4854 | notch 3 | NM_000435.2 |
| NOTCH4 | 4855 | notch 4 | NM_004557.3 |
| NR4A3 | 8013 | nuclear receptor subfamily 4 group A member 3 | NM_006981.3 |
| NRAS | 4893 | neuroblastoma RAS viral oncogene homolog | NM_002524.4 |
| NRG1 | 3084 | neuregulin 1 | NM_013964.3 |
| NSD1 | 64324 | nuclear receptor binding SET domain protein 1 | NM_022455.4 |
| NT5C2 | 22978 | 5'-nucleotidase, cytosolic II | NM_001134373.2 |
| NTRK1 | 4914 | neurotrophic receptor tyrosine kinase 1 | NM_002529.3 |
| NTRK2 | 4915 | neurotrophic receptor tyrosine kinase 2 | NM_006180.3 |
| NTRK3 | 4916 | neurotrophic receptor tyrosine kinase 3 | NM_001012338.2 |
| NUF2 | 83540 | NUF2, NDC80 kinetochore complex component | NM_031423.3 |
| NUP98 | 4928 | nucleoporin 98 | XM_005252950.1 |
| PAK1 | 5058 | p21 | NM_002576.4 |
| PAK5 | 57144 | p21 | NM_177990.2 |
| PAX8 | 7849 | paired box 8 | NM_003466.3 |
| PDCD1 | 5133 | programmed cell death 1 | NM_005018.2 |
| PDCD1LG2 | 80380 | programmed cell death 1 ligand 2 | NM_025239.3 |
| PDGFB | 5155 | platelet derived growth factor subunit B | NM_002608.2 |
| PDGFRA | 5156 | platelet derived growth factor receptor alpha | NM_006206.4 |
| PDGFRB | 5159 | platelet derived growth factor receptor beta | NM_002609.3 |
| PGBD5 | 79605 | piggyBac transposable element derived 5 | NM_001258311.1 |
| PGR | 5241 | progesterone receptor | NM_000926.4 |
| PIK3CA | 5290 | phosphatidylinositol-4,5-bisphosphate 3-kinase catalytic subunit alpha | NM_006218.2 |
| PIK3CB | 5291 | phosphatidylinositol-4,5-bisphosphate 3-kinase catalytic subunit beta | NM_006219.2 |
| PIK3CD | 5293 | phosphatidylinositol-4,5-bisphosphate 3-kinase catalytic subunit delta | NM_005026.3 |
| PIK3CG | 5294 | phosphatidylinositol-4,5-bisphosphate 3-kinase catalytic subunit gamma | NM_002649.2 |
| PLCG1 | 5335 | phospholipase C gamma 1 | NM_182811.1 |
| PLCG2 | 5336 | phospholipase C gamma 2 | NM_002661.3 |
| PPARG | 5468 | peroxisome proliferator activated receptor gamma | NM_015869.4 |
| PPM1D | 8493 | protein phosphatase, Mg2+/Mn2+ dependent 1D | NM_003620.3 |
| PRKACA | 5566 | protein kinase cAMP-activated catalytic subunit alpha | NM_002730.3 |
| PRKCI | 5584 | protein kinase C iota | NM_002740.5 |
| PTPN1 | 5770 | protein tyrosine phosphatase, non-receptor type 1 | NM_001278618.1 |
| PTPN11 | 5781 | protein tyrosine phosphatase, non-receptor type 11 | NM_002834.3 |
| RAB35 | 11021 | RAB35, member RAS oncogene family | NM_006861.6 |
| RAC1 | 5879 | ras-related C3 botulinum toxin substrate 1 | NM_018890.3 |
| RAC2 | 5880 | ras-related C3 botulinum toxin substrate 2 | NM_002872.4 |
| RAF1 | 5894 | Raf-1 proto-oncogene, serine/threonine kinase | NM_002880.3 |
| RBM15 | 64783 | RNA binding motif protein 15 | NM_022768.4 |
| REL | 5966 | REL proto-oncogene, NF-kB subunit | NM_002908.2 |
| RET | 5979 | ret proto-oncogene | NM_020975.4 |
| RHEB | 6009 | Ras homolog enriched in brain | NM_005614.3 |
| RHOA | 387 | ras homolog family member A | NM_001664.2 |
| RICTOR | 253260 | RPTOR independent companion of MTOR complex 2 | NM_152756.3 |
| RIT1 | 6016 | Ras like without CAAX 1 | NM_006912.5 |
| ROS1 | 6098 | ROS proto-oncogene 1, receptor tyrosine kinase | NM_002944.2 |
| RPS6KA4 | 8986 | ribosomal protein S6 kinase A4 | NM_003942.2 |
| RPS6KB2 | 6199 | ribosomal protein S6 kinase B2 | NM_003952.2 |
| RPTOR | 57521 | regulatory associated protein of MTOR complex 1 | NM_020761.2 |
| RRAGC | 64121 | Ras related GTP binding C | NM_022157.3 |
| RRAS | 6237 | related RAS viral | NM_006270.3 |
| RRAS2 | 22800 | related RAS viral | NM_012250.5 |
| RUNX1T1 | 862 | RUNX1 translocation partner 1 | NM_001198626.1 |
| SCG5 | 6447 | secretogranin V | NM_001144757.1 |
| SERPINB3 | 6317 | serpin family B member 3 | NM_006919.2 |
| SETBP1 | 26040 | SET binding protein 1 | NM_015559.2 |
| SETD1A | 9739 | SET domain containing 1A | NM_014712.2 |
| SETDB1 | 9869 | SET domain bifurcated 1 | NM_001145415.1 |
| SF3B 1 | 23451 | splicing factor 3b subunit 1 | NM_012433.2 |
| SFRP2 | 6423 | secreted frizzled related protein 2 | NM_003013.2 |
| SGK1 | 6446 | serum/glucocorticoid regulated kinase 1 | NM_005627.3 |
| SHOC2 | 8036 | SHOC2, leucine rich repeat scaffold protein | NM_007373.3 |
| SMARCE1 | 6605 | SWI/SNF related, matrix associated, actin dependent regulator of chromatin, subfamily e, member 1 | NM_003079.4 |
| SMO | 6608 | smoothened, frizzled class receptor | NM_005631.4 |
| SMYD3 | 64754 | SET and MYND domain containing 3 | NM_001167740.1 |
| SOS1 | 6654 | SOS Ras/Rac guanine nucleotide exchange factor 1 | NM_005633.3 |
| SOX2 | 6657 | SRY-box 2 | NM_003106.3 |
| SOX9 | 6662 | SRY-box 9 | NM_000346.3 |
| SRC | 6714 | SRC proto-oncogene, non-receptor tyrosine kinase | NM_198291.2 |
| SS18 | 6760 | SS18, nBAF chromatin remodeling complex subunit | NM_001007559.2 |
| STAT3 | 6774 | signal transducer and activator of transcription 3 | NM_139276.2 |
| STAT5A | 6776 | signal transducer and activator of transcription 5A | NM_003152.3 |
| STAT5B | 6777 | signal transducer and activator of transcription 5B | NM_012448.3 |
| STAT6 | 6778 | signal transducer and activator of transcription 6 | NM_001178078.1 |
| STK19 | 8859 | serine/threonine kinase 19 | NM_004197.1 |
| SYK | 6850 | spleen associated tyrosine kinase | NM_003177.5 |
| TAL1 | 6886 | TAL bHLH transcription factor 1, erythroid differentiation factor | NM_001287347.2 |
| TCL1A | 8115 | T-cell leukemia/lymphoma 1A | NM_001098725.1 |
| TCL1B | 9623 | T-cell leukemia/lymphoma 1B | NM_004918.3 |
| TERT | 7015 | telomerase reverse transcriptase | NM_198253.2 |
| TFE3 | 7030 | transcription factor binding to IGHM enhancer 3 | NM_006521.5 |
| TLX1 | 3195 | T-cell leukemia homeobox 1 | NM_005521.3 |
| TLX3 | 30012 | T-cell leukemia homeobox 3 | NM_021025.2 |
| TP63 | 8626 | tumor protein p63 | NM_003722.4 |
| TRA | 6955 | T-cell receptor alpha locus | |
| TRB | 6957 | T cell receptor beta locus | |
| TRD | 6964 | T cell receptor delta locus | |
| TRG | 6965 | T cell receptor gamma locus | |
| TRIP13 | 9319 | thyroid hormone receptor interactor 13 | NM_004237.3 |
| TSHR | 7253 | thyroid stimulating hormone receptor | NM_000369.2 |
| TYK2 | 7297 | tyrosine kinase 2 | NM_003331.4 |
| U2AF1 | 7307 | U2 small nuclear RNA auxiliary factor 1 | NM_006758.2 |
| UBR5 | 51366 | ubiquitin protein ligase E3 component n-recognin 5 | NM_015902.5 |
| USP8 | 9101 | ubiquitin specific peptidase 8 | NM_001128610.2 |
| VAV1 | 7409 | vav guanine nucleotide exchange factor 1 | NM_005428.3 |
| VAV2 | 7410 | vav guanine nucleotide exchange factor 2 | NM_001134398.1 |
| VEGFA | 7422 | vascular endothelial growth factor A | NM_001171623.1 |
| WHSC1 | 7468 | Wolf-Hirschhorn syndrome candidate 1 | NM_001042424.2 |
| WT1 | 7490 | Wilms tumor 1 | NM_024426.4 |
| WWTR1 | 25937 | WW domain containing transcription regulator 1 | NM_001168280.1 |
| XBP1 | 7494 | X-box binding protein 1 | NMp_005080.3 |
| XIAP | 331 | X-linked inhibitor of apoptosis | NM_001167.3 |
| XPO1 | 7514 | exportin 1 | NM_003400.3 |
| YAP1 | 10413 | Yes associated protein 1 | NM_001130145.2 |
| YES1 | 7525 | YES proto-oncogene 1, Src family tyrosine kinase | NM_005433.3 |
| YY1 | 7528 | YY1 transcription factor | NM_003403.4 |
| ZBTB20 | 26137 | zinc finger and BTB domain containing 20 | NM_001164342.2 |

The systems and methods described herein provide the unexpected results of improving the use of non-human cell-free nucleic acids for the detection of cancer by removing the requirement for taxonomic assignment of the nucleic acids prior to training of machine learning algorithms. From the perspective of cancer diagnostics, in some embodiments, a sample of cell-free nucleic acid may, in view of taxonomy classification, comprise five major groups of nucleic acids: (1) nucleic acids from host mammalian cells that do not bear any mutations of oncological significance; (2) nucleic acids from host mammalian cells that do bear mutations of oncological significance; (3) microbial nucleic acids derived from known microbes; (4) microbial nucleic acids derived from unknown microbes (i.e., those microbes for which annotated reference genomes do not yet exist); and (5) unidentified nucleic acids (i.e., nucleic acids that do not map to any known reference genome). Hitherto, machine learning classification of cancers based on a subject's cell-free non-human nucleic acids has been restricted to utilizing non-human sequencing reads that can be assigned to a defined microbial taxonomy, thereby dispensing with the data content represented in the unassigned sequence reads (the aforementioned groups 4 and 5). For example, in Poore et al. (Nature. 2020 Mar;579(7800):567-574 and WO2020093040A1), the cancer-specific abundance of microbial nucleic acids present in a sample are used to form a diagnosis of disease. This method relies upon first determining the genus-level taxonomic identity of non-human sequencing reads via fast k-mer mapping to a database of microbial reference genomes using Kraken, a requirement that leads to > 90% of all non-human sequencing reads being discarded from the analysis as shown in **Table 3.** This loss of data is an unavoidable consequence that existing reference databases only represent a small fraction of the total microbes present in a metagenomic sample, such as the plasma samples analyzed in **Table 3.** To capture the loss of data, the methods and systems described herein may incorporate all non-human sequencing reads into the training of the machine learning algorithms by way of a reference-free analysis of k-mer content. (Here, 'reference-free' refers to a process of nucleic acid analysis that explicitly does not utilize reference genomes to make taxonomic assignments.)

**Table 3 Percentage of unassigned non-human sequencing reads in Poore et al.**

| **Sample ID** | **# Assigned non-human reads** | **# Unassigned non-human reads** | **Total non-human reads** | **% Unassigned non-human reads** |
|---|---|---|---|---|
| HNL8 | 8042 | 110160 | 118202 | 93.20% |
| HNN1 | 7620 | 112785 | 120405 | 93.67% |
| LC20 | 5644 | 91631 | 97275 | 94.20% |
| LC4 | 6342 | 92838 | 99180 | 93.61% |
| PC1 | 6806 | 105669 | 112475 | 93.95% |
| PC17 | 7160 | 88246 | 95406 | 92.50% |
| PC2 | 6512 | 116099 | 122611 | 94.69% |
| PC30 | 6789 | 107804 | 114593 | 94.08% |
| PC39 | 3330 | 48969 | 52299 | 93.63% |

The systems and methods disclosed herein may comprise a method of computationally segregating and/or separating subjects' nucleic acid sequencing reads into reference-mappable nucleic acid sequencing reads and non-reference mappable nucleic acid sequencing reads prior to further analysis e.g., generating nucleic acid k-mers and/or training predictive models. In some cases, reference-mappable sequencing reads may comprise human and/or non-human nucleic acid sequencing reads that map to a human and/or non-human reference genome database. In some cases, mappable sequencing reads may comprise nucleic acid sequencing reads of non-human (e.g., microbial, viral, fungal, archael, etc.), human, somatic human mutated, or any combination thereof nucleic acid sequencing reads. In some cases, non-reference mappable nucleic acid sequencing reads may comprise nucleic acid sequencing reads that did not map to microbial, human, or human cancerous genomic databases. In some cases, non-reference mappable sequencing may comprise dark-matter reads.

In some instances, the methods described elsewhere herein, may utilize computationally deconstructed non-human, somatic human mutated, non-reference mappable, or any combination thereof nucleic sequencing reads into a collection of k-mers of a defined k-mer base pair length k that can be grouped and/or counted to produce k-mer abundances as inputs for machine learning algorithms.

In some embodiments, the k-mer base pair length may be about 20 base pairs to about 35 base pairs. In some embodiments, the k-mer base pair length may be about 20 base pairs to about 22 base pairs, about 20 base pairs to about 24 base pairs, about 20 base pairs to about 26 base pairs, about 20 base pairs to about 28 base pairs, about 20 base pairs to about 30 base pairs, about 20 base pairs to about 32 base pairs, about 20 base pairs to about 35 base pairs, about 22 base pairs to about 24 base pairs, about 22 base pairs to about 26 base pairs, about 22 base pairs to about 28 base pairs, about 22 base pairs to about 30 base pairs, about 22 base pairs to about 32 base pairs, about 22 base pairs to about 35 base pairs, about 24 base pairs to about 26 base pairs, about 24 base pairs to about 28 base pairs, about 24 base pairs to about 30 base pairs, about 24 base pairs to about 32 base pairs, about 24 base pairs to about 35 base pairs, about 26 base pairs to about 28 base pairs, about 26 base pairs to about 30 base pairs, about 26 base pairs to about 32 base pairs, about 26 base pairs to about 35 base pairs, about 28 base pairs to about 30 base pairs, about 28 base pairs to about 32 base pairs, about 28 base pairs to about 35 base pairs, about 30 base pairs to about 32 base pairs, about 30 base pairs to about 35 base pairs, or about 32 base pairs to about 35 base pairs. In some embodiments, the k-mer base pair length may be about 20 base pairs, about 22 base pairs, about 24 base pairs, about 26 base pairs, about 28 base pairs, about 30 base pairs, about 32 base pairs, or about 35 base pairs. In some embodiments, the k-mer base pair length may be at least about 20 base pairs, about 22 base pairs, about 24 base pairs, about 26 base pairs, about 28 base pairs, about 30 base pairs, or about 32 base pairs. In some embodiments, the k-mer base pair length may be at most about 22 base pairs, about 24 base pairs, about 26 base pairs, about 28 base pairs, about 30 base pairs, about 32 base pairs, or about 35 base pairs.

In some embodiments, the training data for the predictive models and/or machine learning algorithms may comprise all or a subset of k-mers, described elsewhere herein. For example, assuming a read length *L* of 150 base pairs and a k-mer of length *k* of 31 base pairs, 120 unique k-mers (*L - k +* 1) may be produced from each sequencing read; using the data from **Table 3** as a point of reference, the disclosed reference-free, k-mer based approach, in some embodiments may yield an average of 15-fold more sequencing data (> 12.4 x 10⁶ non-human k-mers) available for machine learning analysis compared to a restricted analysis of only those reads with assigned taxonomies. In this regard, the methods of this invention, in some embodiments, may provide a complete representation of nucleic acid sequences that can be analyzed to find cancer-specific/characteristic features.

The description provided herein discloses methods that may utilize nucleic acids of non-human origin to diagnose a condition (i.e., cancer). In some embodiments, the disclosed invention may provide better than expected clinical outcomes compared to a typical pathology report as it is not necessary to include one or more of observed tissue structure, cellular atypia, or other subjective measures traditionally used to diagnose cancer. In some embodiments, the disclosed methods may provide a high degree of sensitivity of detecting and/or diagnosing cancer of a subject by combining data from both sequencing reads of oncological significance with the non-human reads rather than just modified human (i.e., cancerous) sources, which are modified often at extremely low frequencies in a background of 'normal' human sources. In some embodiments, the methods disclosed herein may achieve such outcomes by either solid tissue or liquid (e.g., blood, sputum, urine, etc.) biopsy samples, the latter of which requires minimal sample preparation and is minimally invasive. In some embodiments, the methods of the disclosure herein that may determine or diagnose cancer of an individual from a liquid biopsy-based samples may overcome challenges posed by circulating tumor DNA (ctDNA) assays, which often suffer from sensitivity issues due to cell-free DNA (cfDNA) that originates from non-malignant human cells. In some embodiments, the disclosed method may comprise an assay that may distinguish between cancer types, which ctDNA assays typically are not able to achieve, since most common cancer genomic aberrations are shared between cancer types (e.g., TP53 mutations, KRAS mutations).

In some embodiments, the methods disclosed herein may comprise a method of training a predictive model configured to diagnose or determine the presence or lack thereof cancer of subjects. In some instances, the predictive model may comprise one or more machine learning algorithms. In some cases, the predictive model may be trained with human somatic mutations and k-mer nucleic acid signatures, described elsewhere herein. In some cases, the human somatic mutations and k-mer nucleic acid signatures may comprise nucleic acid sequences provided by real-time sequencing data, retrospective sequencing data or any combination thereof sequencing data. In some embodiments, real-time sequencing data may comprise sequencing data that is obtained and analyzed prospectively for the presence or lack thereof cancer. In some embodiments, retrospective sequencing data may comprise sequencing data that has been collected in the past and is retrospectively analyzed. In some embodiments, the human somatic mutations and non-human k-mers may comprise combination signatures.

The disclosure provided herein also describes a method of diagnosing and/or determine the presence or lack thereof cancer of subjects. In some instances, the method may comprise: (a) taking a blood sample from a subject during a routine clinic visit; (b) preparing plasma or serum from that blood sample, extracting the nucleic acids contained within, and amplifying the sequences for specific combination signatures determined previously, by way of the previously trained predictive models, to be useful features for diagnosing cancer; (c) obtaining a digital readout of the presence and/or abundance of the combination signatures (e.g., human somatic mutated and k-mer nucleic acid prevalence and/or abundances); (d) normalizing the presence and/or abundance data on an adjacent computer or cloud computing infrastructure and inputting it into a previously trained machine learning model; (e) reading out a prediction and a degree of confidence for how likely this sample: (1) is associated with the presence or absence of cancer, (2) is associated with cancer of a particular type or bodily location, or (3) is associated with a high, intermediate, or low likelihood of response to a range of cancer therapies; and (f) using the sample's somatic mutation and non-human k-mer information to continue training the machine learning model if additional information is later inputted by the user.

The method of diagnosing cancer of a subject according to the present invention is defined in the claims and comprises: (a) determining a plurality of somatic mutations and non-human k-mer sequences of a subject's sample; (b) comparing the plurality of somatic mutations and the plurality of non-human k-mer sequences of the subject with a plurality of somatic mutations and non-human k-mer sequences for a given cancer; and (c) diagnosing cancer of the subject by providing a probability of the presence or lack thereof cancer based at least in part on the comparison of the subject's plurality of somatic mutations and non-human k-mer sequences for the given cancer. In some cases, determining the plurality of somatic mutation may further comprises counting somatic mutations of the subject's sample. In some instances, determining the plurality of non-human k-mer sequences may comprise counting the non-human k-mer sequences of the subject's sample. In some cases, diagnosing the cancer of the subject may further comprise determining a category or location of the cancer. In some instances, diagnosing the cancer of the subject may further comprise determining one or more types of the subject's cancer. In some cases, diagnosing the cancer of the subject may further comprise determining one or more subtypes of the subject's cancer. In some instances, diagnosing the cancer of the subject may further comprise determining the stage of the subject's cancer, cancer prognosis, or any combination thereof. In some cases, diagnosing the cancer of the subject may further comprise determining a type of cancer at a low-stage. In some cases, the type of cancer at low stage may comprise stage I, or stage II cancers. In some instances, diagnosing the cancer of the subject may further comprise determining the mutation status of the subject's cancer. In some instances, diagnosing the cancer of the subject may further comprise determining the subject's response to therapy to treat the subject's cancer. In some instances, the cancer may comprise: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof. In some cases, the subject may be a non-human mammal. In some instances, the subject may be a human. In some cases, the subject may be a mammal. In some instances, the plurality of non-human k-mer sequences may originate from the following non-mammalian domains of life: viral, bacterial, archaeal, fungal, or any combination thereof.

The disclosure provided herein also describes a method of diagnosing cancer of a subject using a trained predictive model. In some cases, the method may comprise: (a) receiving a plurality of somatic mutations and non-human k-mer nucleic acid sequences of a first one or more subjects' nucleic acid samples; (b) providing as an input to a trained predictive model the first subjects' plurality of somatic mutations and non-human k-mer nucleic acid sequences, wherein the trained predictive model is trained with a second one or more subjects' plurality of somatic mutation nucleic acid sequences, non-human k-mer nucleic acid sequences, and corresponding clinical classifications of the second one or more subjects', and wherein the first one or more subjects and the second one or more subjects are different subjects; and (c) diagnosing cancer of the first one or more subjects based at least in part on an output of the rained predictive model. In some cases, receiving the plurality of somatic mutation nucleic acid sequences may further comprises counting somatic mutation nucleic acid sequences of the first one or more subjects' nucleic acid samples. In some instances, receiving the plurality of non-human k-mer nucleic acid sequences may further comprise counting the non-human k-mer nucleic acid sequences of the first one or more subjects' nucleic acid samples. In some cases, diagnosing the cancer of the first one or more subjects may further comprise determining a category or location of the first one or more subjects' cancers. In some instances, diagnosing the cancer of the first one or more subjects may further comprise determining one or more types of the first one or more subjects' cancer. In some cases, diagnosing the cancer of the first one or more subjects may further comprise determining one or more subtypes of the first one or more subjects' cancers. In some instances, diagnosing the cancer of the first one or more subjects may further comprise determining the first one or more subjects' stage of cancer, cancer prognosis, or any combination thereof. In some cases, diagnosing the cancer of the first one or more subjects may further comprise determining a type of cancer at a low-stage. In some cases, the type of cancer at low stage may comprise stage I, or stage II cancers. In some instances, diagnosing the cancer of the first one or more subjects may further comprise determining the mutation status of the first one or more subjects' cancers. In some instances, diagnosing the cancer of the first one or more subjects may further comprise determining the first one or more subjects' response to therapy to treat the first one or more subjects' cancers. In some instances, the cancer may comprise: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof. In some cases, the first one or more subjects and second one or more subjects may be a non-human mammal. In some instances, the first one or more subjects and second one or more subjects may be a human. In some cases, the first one or more subjects may be a mammal. In some instances, the plurality of non-human k-mer sequences may originate from the following non-mammalian domains of life: viral, bacterial, archaeal, fungal, or any combination thereof.

The disclosure provided herein also describes a method to generate a trained predictive model configured to diagnose and/or determine the presence or lack thereof cancer of a subject. In some cases, the method may comprise: (a) sequencing the nucleic acid content of subjects' liquid biopsy sample; and (b) generating a diagnostic model by training the diagnostic model with the sequenced nucleic acids of the subjects. In some cases, the sequencing method may comprise next-generation sequencing, long-read sequencing (e.g., nanopore sequencing) or any combination thereof. In some cases, the diagnostic model **118** may comprise a trained machine learning algorithm **117** as shown in **FIG. 1C****.** In some cases, the diagnostic model may comprise a regularized machine learning model. In some cases, the trained machine learning model algorithm may comprise a linear regression, logistic regression, decision tree, support vector machine (SVM), naive bayes, k-nearest neighbors (kNN), k-Means, random forest model, or any combination thereof.

The disclosure provided herein also describes a method of training a machine learning algorithm, as seen in **FIGS. 1A-1C****.** In some instances, the machine learning algorithm **117** may be trained with next generation sequencing (NGS) reads **103** comprising nucleic acid sequencing data derived from nucleic acids from a plurality of known healthy subjects **101** and a plurality of known cancer subjects **102.** In some cases, the machine learning algorithm **117** may be trained with nucleic acid sequencing data **103** that has been processed through a bioinformatics pipeline. In some cases, the bioinformatics pipeline may comprise: (a) computationally filtering all sequencing reads mapping to the human genome using fast k-mer mapping with exact matching **104;** (b) discarding all exact matches to the human reference genome **105;** (c) processing the remaining reads **106,** where the remaining reads may comprise human reads that do not map exactly to the reference genome and are likely enriched for somatic mutations of oncological significance (hereinafter 'somatic mutations') and reads from known microbes, reads from unknown microbes, unidentified reads, or any combination thereof; (d) decontaminating DNA contaminants through a decontamination pipeline **107** to remove sequences derived from common microbial contaminants, thereby producing a set of *in silico* decontaminated reads **108;** (e) performing a second round of mapping to the human reference genome via bowtie 2 **109** to obtain somatic human mutated sequences (inexact matches to the human reference genome) **110** and non-human sequences **113;** (f) querying a cancer mutation database **111** with the collection of somatic human mutated sequences **110** to identify known cancer mutations; (g) generating an abundance of the somatic human mutated sequences **112;** (h) deconstructing the non-human sequence reads **113** into a collection of k-mers **114;** (i) analyzing the k-mers to produce k-mer identities and abundance **115;** (j) combining the somatic human mutation sequence abundance data **112** and the k-mer identity and abundance data **115** to produce a machine learning training dataset **116.** K-mer analysis may be accomplished with the programs Jellyfish, UCLUST, GenomeTools (Tallymer), KMC2, DSK, Gerbil or any equivalent thereof. In some cases, k-mer analysis may comprise counting the k-mers and organizing the k-mers by identity into an abundance table. In some cases, the human reference genome may comprise GRCh38. In some cases, the abundance of the somatic human mutated sequences may be organized in an abundance table. In some instances, the fast k-mer mapping with exact matching may be completed with Kraken software package against GRCh38 human genome database.

In some cases, the machine learning algorithm **117** may be trained with the machine learning training dataset **116** resulting in a trained diagnostic model **118,** where the trained diagnostic model may determine nucleic acid signatures associated with and/or indicative of healthy subjects **119** and nucleic acid signatures associated with/indicative of subjects with cancer **120.**

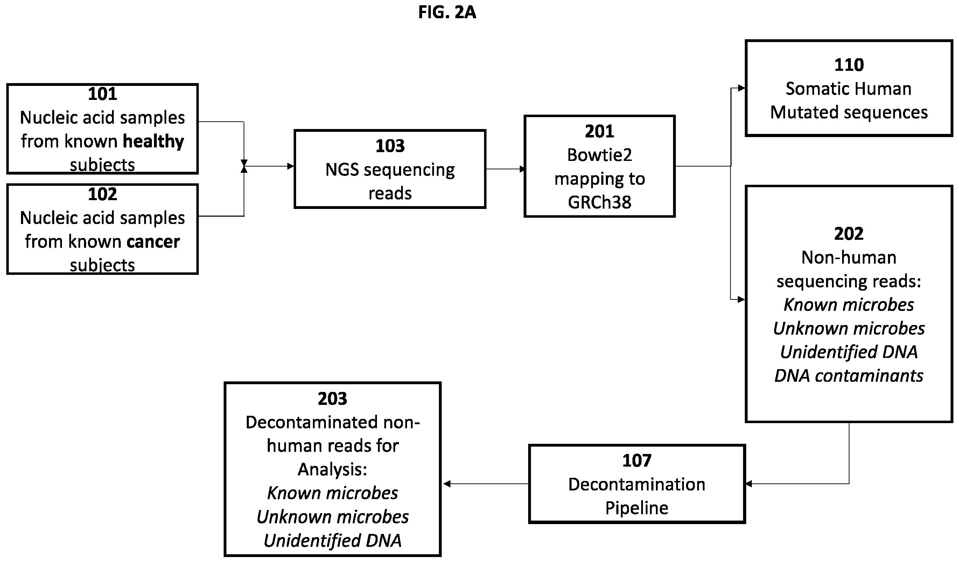

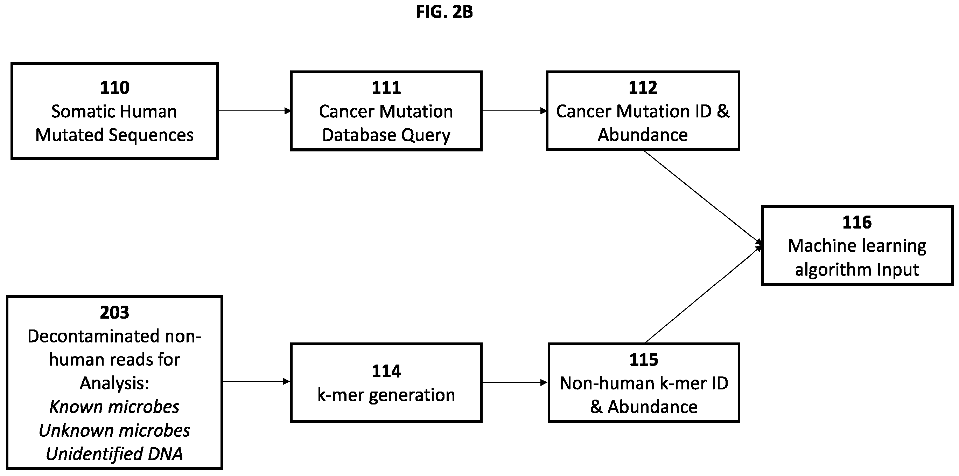

In some instances, the methods of the disclosure provided herein may comprise a method of training a machine learning algorithm, as seen in **FIGS. 2A-2B****.** In some cases, the method may comprise: (a) providing nucleic acid samples from known healthy subjects **101** and nucleic acid samples from known cancer subjects **102;** (b) sequencing the nucleic acid samples of the known healthy subjects and the known cancer subjects thereby producing a plurality of sequencing reads **103;** (c) mapping the sequencing reads to a human genome database thereby separating the sequencing reads into somatic human mutated sequencing reads **110** and non-human sequencing reads **202;** (d) decontaminating the non-human sequencing reads **107** thereby producing a plurality of decontaminated non-human sequencing reads **203;** (e) querying the somatic human mutated sequencing reads **110** against a cancer mutation database **111** thereby producing a plurality of cancer mutation ID & abundance **112** from the somatic human mutated sequencing reads; (f) generating a plurality of k-mers **114** and associated non-human k-mer ID and abundance **115** from the from the decontaminated non-human reads **203;** (g) combining the non-human k-mer IDs and abundances and the plurality of somatic human mutated sequences ID and abundances into a machine learning training dataset **116;** and (f) training a machine learning algorithm **117** with the machine learning training dataset **116** thereby producing a trained diagnostic machine learning model **118.** In some instances, the trained diagnostic machine learning model may comprise a machine learning healthy signature **119,** cancer signature **120,** or any combination thereof signatures. In some cases, mapping the sequencing reads to a human genome database may be accomplished using Bowtie 2. In some instances, the human genome database may comprise GRCh38. In some cases, the non-human sequencing reads may comprise sequencing reads of known microbes, unknown microbes, unidentified DNA, DNA contaminants, or any combination thereof.

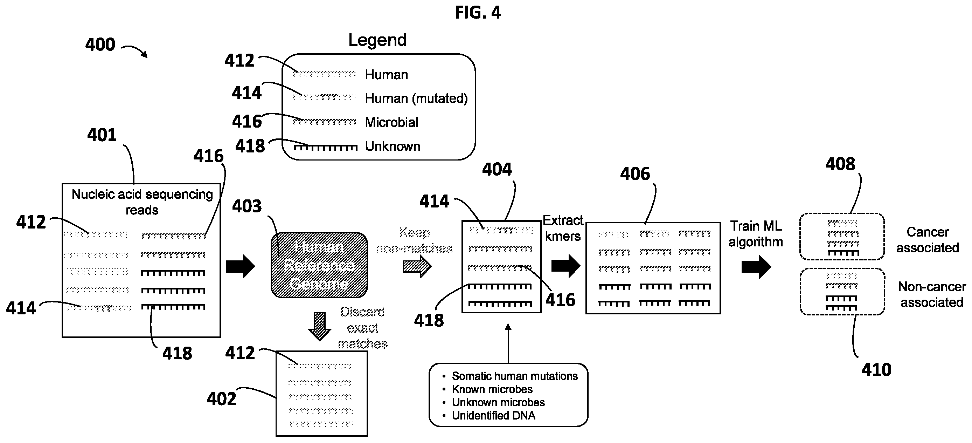

The disclosure provided herein also describes a method of generating predictive cancer model **400,** as seen in **FIG. 4****.** In some cases, the method may comprise: (a) providing one or more nucleic acid sequencing reads of one or more subjects' biological samples **401;** (b) filtering the one or more nucleic acid sequencing reads with a human genome database **403** thereby producing one or more filtered sequencing reads **404;** (c) generating a plurality of k-mers from the one or more filtered sequencing reads **406;** and (d) generating a predictive cancer model by training a predictive model with the plurality of k-mers and corresponding clinical classification of the one or more subjects (**408, 410**). In some cases, the trained predictive model may comprise a set of cancer associated k-mers **408.** In some cases, the one or more sequencing reads may comprise human **412,** human somatic mutated **414,** microbial **416,** non-human non-reference mappable (i.e., "unknown") **418,** or any combination thereof sequencing reads. In some instances, the trained predictive model may comprise a set of non-cancer associated k-mers **410.** In some cases, the method may further comprise determining an abundance of the plurality of k-mers and training the predictive model with the abundance of the plurality of k-mers. In some cases, filtering may be performed by exact matching between the one or more nucleic acid sequencing reads and the human reference genome database. In some instances, exact matching may comprise computationally filtering of the one or more nucleic acid sequencing reads with the software program Kraken or Kraken 2. In some cases, exact matching may comprise computationally filtering of the one or more nucleic acid sequencing reads with the software program bowtie 2 or any equivalent thereof. In some cases, the method may further comprise performing in-silico decontamination of the one or more filtered sequencing reads thereby producing one or more decontaminated sequencing reads. In some instances, the in-silico decontamination may identify and remove non-human contaminant features, while retaining other non-human signal features. In some cases, the method may further comprise mapping the one or more decontaminated sequencing reads to a build of a human reference genome database to produce a plurality of mutated human sequence alignments. In some instances, the human reference genome database may comprise GRCh38. In some instances, mapping may be performed by bowtie 2 sequence alignment tool or any equivalent thereof. In some cases, mapping may comprise end-to-end alignment, local alignment, or any combination thereof. In some instances, the method may further comprise identifying cancer mutations in the plurality of mutated human sequence alignments by querying a cancer mutation database. In some instances the cancer mutation database may be derived from the Catalogue of Somatic Mutations in Cancer (COSMIC), the Cancer Genome Project (CGP), The Cancer Genome Atlas (TGCA), the International Cancer Genome Consortium (ICGC) or any combination thereof. In some cases, the method may further comprise generating a cancer mutation abundance table with the cancer mutations. In some instances, the plurality of k-mers may comprise non-human k-mers, human mutated k-mers, non-classified DNA k-mers, or any combination thereof. In some instances, the non-human k-mers may originate from the following domains of life: bacterial, archaeal, fungal, viral, or any combination thereof. In some cases, the one or more biological samples may comprise a tissue sample, a liquid biopsy sample, or any combination thereof. In some cases, the liquid biopsy may comprise: plasma, serum, whole blood, urine, cerebral spinal fluid, saliva, sweat, tears, exhaled breath condensate, or any combination thereof. In some instances, the one or more subjects may be human or non-human mammal. In some cases, the one or more nucleic acid sequencing reads may comprise DNA, RNA, cell-free DNA, cell-free RNA, exosomal DNA, exosomal RNA, circulating tumor cell DNA, circulating tumor cell RNA, or any combination thereof. In some instances, the output of the predictive cancer model may provide a diagnosis of a presence or absence of cancer, a cancer body site location, cancer somatic mutations, or any combination thereof associated with the presence or absence of cancer of a subjects. In some cases, the output of the predictive cancer model may comprise an analysis of the cancer somatic mutations, the abundance of the plurality of k-mers, or any combination thereof. In some instances, the trained predictive model may be trained with a set of cancer mutation and k-mer abundances that are known to be present or absent with a characteristic abundance in a cancer of interest. In some cases, the predictive cancer model may be configured to determine the presence or lack thereof one or more types of cancer of a subject. In some instances, the one or more types of cancer may be at a low-stage. In some cases, the low-stage may comprise stage I, stage II, or any combination thereof stages of cancer. In some instances, the predictive cancer model may be configured to determine the presence or lack thereof one or more subtypes of cancer of a subject. In some cases, the predictive cancer model may be configured to predict a stage of cancer, predict cancer prognosis, or any combination thereof. In some instances, the predictive cancer model may be configured to predict a therapeutic response of a subject when administered a therapeutic compound to treat the subject's cancer. In some cases, the predictive cancer model may be configured to determine an optimal therapy to treat a subject's cancer. In some instances, the predictive cancer model may be configured to longitudinally model a course of a subject's one or more cancers' response to a therapy, thereby producing a longitudinal model of the course of the subjects' one or more cancers' response to therapy. In some cases, the predictive cancer model may be configured to determine an adjustment to the course of therapy of the subject's one or more cancers based at least in part on the longitudinal model. In some instances, the predictive cancer model may be configured to determine the presence or lack thereof: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof cancer of a subject. In some cases, determining the abundance of the plurality of k-mers may be performed by Jellyfish, UCLUST, GenomeTools (Tallymer), KMC2, Gerbil, DSK, or any combination thereof. In some instances, the clinical classification of the one or more subjects may comprise healthy, cancerous, non-cancerous disease, or any combination thereof. In some cases, the one or more filtered sequencing reads may comprise non-human sequencing reads, non-matched non-human sequencing reads, or any combination thereof. IN some instances, the non-matched non-human sequencing reads may comprise sequencing reads that do not match to a non-human reference genome database.

The disclosure provided herein also describes a method of generating predictive cancer model. In some cases, the method may comprise: (a) sequencing nucleic acid compositions of one or more subjects' biological samples thereby generating one or more sequencing reads; (b) filtering the one or more nucleic acid sequencing reads with a human genome database thereby producing one or more filtered sequencing reads; (c) generating a plurality of k-mers from the one or more filtered sequencing reads; and (d) generating a predictive cancer model by training a predictive model with the plurality of k-mers and corresponding clinical classification of the one or more subjects. In some cases, the trained predictive model may comprise a set of cancer associated k-mers. In some instances, the trained predictive model may comprise a set of non-cancer associated k-mers. In some cases, the method may further comprise determining an abundance of the plurality of k-mers and training the predictive model with the abundance of the plurality of k-mers. In some cases, filtering may be performed by exact matching between the one or more sequencing reads and the human reference genome database. In some instances, exact matching may comprise computationally filtering of the one or more sequencing reads with the software program Kraken or Kraken 2. In some cases, exact matching may comprise computationally filtering of the one or more sequencing reads with the software program bowtie 2 or any equivalent thereof. In some cases, the method may further comprise performing in-silico decontamination of the one or more filtered sequencing reads thereby producing one or more decontaminated sequencing reads. In some instances, the in-silico decontamination may identify and remove non-human contaminant features, while retaining other non-human signal features. In some cases, the method may further comprise mapping the one or more decontaminated sequencing reads to a build of a human reference genome database to produce a plurality of mutated human sequence alignments. In some instances, the human reference genome database may comprise GRCh38. In some instances, mapping may be performed by bowtie 2 sequence alignment tool or any equivalent thereof. In some cases, mapping may comprise end-to-end alignment, local alignment, or any combination thereof. In some instances, the method may further comprise identifying cancer mutations in the plurality of mutated human sequence alignments by querying a cancer mutation database. In some instances the cancer mutation database may be derived from the Catalogue of Somatic Mutations in Cancer (COSMIC), the Cancer Genome Project (CGP), The Cancer Genome Atlas (TGCA), the International Cancer Genome Consortium (ICGC) or any combination thereof. In some cases, the method may further comprise generating a cancer mutation abundance table with the cancer mutations. In some instances, the plurality of k-mers may comprise non-human k-mers, human mutated k-mers, non-classified DNA k-mers, or any combination thereof. In some instances, the non-human k-mers may originate from the following domains of life: bacterial, archaeal, fungal, viral, or any combination thereof. In some cases, the one or more biological samples may comprise a tissue sample, a liquid biopsy sample, or any combination thereof. In some cases, the liquid biopsy may comprise: plasma, serum, whole blood, urine, cerebral spinal fluid, saliva, sweat, tears, exhaled breath condensate, or any combination thereof. In some instances, the one or more subjects may be human or non-human mammal. In some cases, the nucleic acid composition may comprise DNA, RNA, cell-free DNA, cell-free RNA, exosomal DNA, exosomal RNA, circulating tumor cell DNA, circulating tumor cell RNA, or any combination thereof. In some instances, the output of the predictive cancer model may provide a diagnosis of a presence or absence of cancer, a cancer body site location, cancer somatic mutations, or any combination thereof associated with the presence or absence of cancer of a subject. In some cases, the output of the predictive cancer model may comprise an analysis of the cancer somatic mutations, the abundance of the plurality of k-mers, or any combination thereof. In some instances, the trained predictive model may be trained with a set of cancer mutation and k-mer abundances that are known to be present or absent with a characteristic abundance in a cancer of interest. In some cases, the predictive cancer model may be configured to determine a presence or lack thereof one or more types of cancer of the subject. In some instances, the one or more types of cancer may be at a low-stage. In some cases, the low-stage may comprise stage I, stage II, or any combination thereof stages of cancer. In some instances, the predictive cancer model may be configured to determine the presence or lack thereof one or more subtypes of cancer of the subjects. In some cases, the predictive cancer model may be configured to predict a subject's a stage of cancer, predict cancer prognosis, or any combination thereof. In some instances, the predictive cancer model may be configured to predict a therapeutic response of a subject when administered a therapeutic compound to treat the subject's cancer. In some cases, the predictive cancer model may be configured to determine an optimal therapy to treat a subject's cancer. In some instances, the predictive cancer model may be configured to longitudinally model a course of a subject's one or more cancers' response to a therapy, thereby producing a longitudinal model of the course of the subjects' one or more cancers' response to therapy. In some cases, the predictive cancer model may be configured to determine an adjustment to the course of therapy of the subject's one or more cancers based at least in part on the longitudinal model. In some instances, the predictive cancer model may be configured to determine the presence or lack thereof: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof cancer of the subject. In some cases, determining the abundance of the plurality of k-mers may be performed by Jellyfish, UCLUST, GenomeTools (Tallymer), KMC2, Gerbil, DSK, or any combination thereof. In some instances, the clinical classification of the one or more subjects may comprise healthy, cancerous, non-cancerous disease, or any combination thereof classifications. In some cases, the one or more filtered sequencing reads may comprise non-human sequencing reads, non-matched non-human sequencing reads, or any combination thereof. In some cases, the one or more filtered sequencing reads may comprise non-exact matches to a reference human genome, non-human sequencing reads, non-matched non-human sequencing reads, or any combination thereof. In some instances, the non-matched non-human sequencing reads may comprise sequencing reads that do not match to a non-human reference genome database.

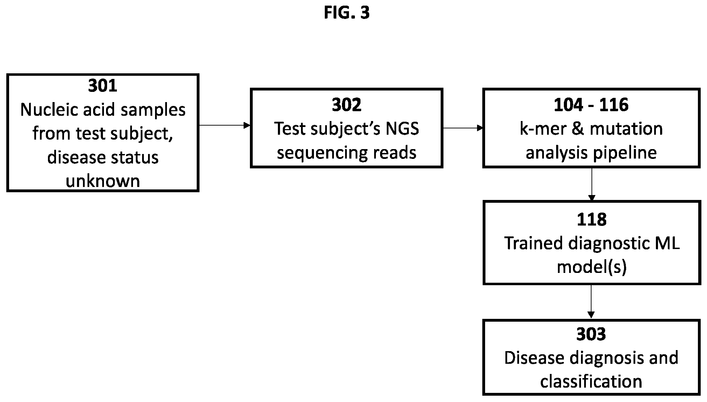

In some cases, the trained diagnostic model **118** may be used to analyze the nucleic acid samples from subjects of unknown disease status **301** and provide a diagnosis of disease and, where applicable, classification of the state of that disease **303,** as seen in **FIG. 3****.**

In some cases, the machine learning algorithm **117** may be trained with nucleic acid sequencing data **103** that has been processed through a bioinformatics pipeline comprising: (a) computationally filtering all sequencing reads mapping to the human genome using bowtie 2 **201;** (b) retaining all inexact matches to the human reference genome comprising mutated human sequences **110;** (c) processing the remaining reads **202,** comprising reads from known microbes, reads from unknown microbes, unidentified reads, DNA contaminants or any combination thereof through a decontamination pipeline **107** to remove sequences derived from common microbial contaminants, thereby producing a set of *in silico* decontaminated reads **203;** (d) querying a cancer mutation database **111** with the collection of somatic human muted sequences **110** to identify known cancer mutations and generate an abundance table of said mutations **112;** (e) deconstructing the non-human sequence reads **203** into a collection of k-mers **114;** (g) counting the k-mers to produce a table of k-mer identities and abundance **115;** (h) combining the somatic human mutation abundance data **112** and the k-mer abundance data **115** to produce a machine learning training dataset **116.** In some cases, k-mer counting may be accomplished with the programs Jellyfish, UCLUST, GenomeTools (Tallymer), KMC2, DSK, Gerbil or any equivalent thereof. The use of these bioinformatics pipelines and databases is not intended to be limiting but to serve as illustrations of the computational means by which one of ordinary skill in the art may arrive at somatic mutation and k-mer abundance data and therefore includes the use of any substantial equivalent to the aforementioned bioinformatics methods and programs.

The disclosure provided herein also describes a method of training a diagnostic model (**FIGS. 1A-1C**) comprising: (a) providing as a training data set (i) one or more subjects' one or more somatic mutation and non-human k-mer abundances **116;** (b) providing as a test set (i) one or more subjects' one or more somatic mutation and non-human k-mer abundances **116;** (c) training the diagnostic model on a 60 to 40 sample ratio of training to validation samples, respectively; and (d) evaluating the diagnostic accuracy of the diagnostic model.

The diagnosis made by the trained diagnostic model may comprise a machine learning signature indicative of a healthy (i.e., cancer-free) subject **119,** or a machine learning derived signature indicative of cancer-positive subject **120** as seen in **FIG. 1C****.** The trained diagnostic model may identify and remove the one more microbial or non-microbial nucleic acids classified as noise while selectively retaining other one or more microbial or non-microbial sequences termed signal.

### Computer Systems

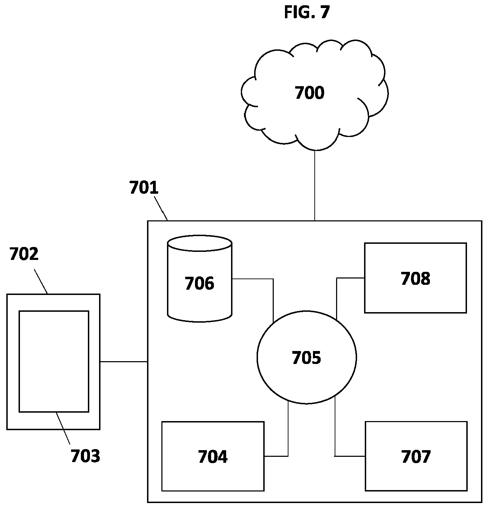

**FIG. 7** shows a computer system **701** suitable for implementing and/or training the models and/or predictive models described herein. The computer system **701** may process various aspects of information of the present disclosure, such as, for example, the one or more subjects' nucleic acid composition sequencing reads. In some cases, the computer system may process the one or more subjects' nucleic acid composition sequencing reads by mapping and/or filtering the sequencing reads against known libraries of genomic sequences for human and/or non-human genomes. In some instances, the computer system may generate one or more k-mer sequences from the human and/or non-human genomes. In some cases, the computer system may be configured to determine an abundance, or a prevalence of a given k-mer sequence, cancer mutation, or any combination thereof, present in the one or more subjects' nucleic acid composition sequencing reads. In some instances, the computer system may prepare k-mer sequence abundances, cancer mutation abundance, and corresponding one or more subjects' clinical classification datasets to be used in training one or more predictive models, where the predictive model may comprise machine learning algorithms. The computer system **701** may be an electronic device. The electronic device may be a mobile electronic device.

The systems disclosed herein may implement one or more predictive models. In some cases, the one or more predictive models may comprise one or more machine learning algorithm configured to determine the presence or lack thereof cancer of one or more subjects based upon their respective k-mer sequences and/or cancer mutation sequence abundances, described elsewhere herein.

In some cases, machine learning algorithms may need to extract and draw relationships between features as conventional statistical techniques may not be sufficient. In some cases, machine learning algorithms may be used in conjunction with conventional statistical techniques. In some cases, conventional statistical techniques may provide the machine learning algorithm with preprocessed features.

In some embodiments, the machine learning algorithm may comprise, for example, an unsupervised learning algorithm, supervised learning algorithm, or any combination thereof. The unsupervised learning algorithm may be, for example, clustering, hierarchical clustering, k-means, mixture models, DBSCAN, OPTICS algorithm, anomaly detection, local outlier factor, neural networks, autoencoders, deep belief nets, hebbian learning, generative adversarial networks, self-organizing map, expectation-maximization algorithm (EM), method of moments, blind signal separation techniques, principal component analysis, independent component analysis, non-negative matrix factorization, singular value decomposition, or a combination thereof. The supervised learning algorithm may be, for example, support vector machines, linear regression, logistic regression, linear discriminant analysis, decision trees, k-nearest neighbor algorithm, neural networks, similarity learning, or a combination thereof. In some embodiments, the machine learning algorithm may comprise a deep neural network (DNN). The deep neural network may comprise a convolutional neural network (CNN). The CNN may be, for example, U-Net, ImageNet, LeNet-5, AlexNet, ZFNet, GoogleNet, VGGNet, ResNet18 or ResNet, etc. Other neural networks may be, for example, deep feed forward neural network, recurrent neural network, LSTM (Long Short Term Memory), GRU (Gated Recurrent Unit), Auto Encoder, variational autoencoder, adversarial autoencoder, denoising auto encoder, sparse auto encoder, boltzmann machine, RBM (Restricted BM), deep belief network, generative adversarial network (GAN), deep residual network, capsule network, or attention/transformer networks, etc.

In some instances, the machine learning algorithm may comprise clustering, scalar vector machines, kernel SVM, linear discriminant analysis, Quadratic discriminant analysis, neighborhood component analysis, manifold learning, convolutional neural networks, reinforcement learning, random forest, Naive Bayes, gaussian mixtures, Hidden Markov model, Monte Carlo, restrict Boltzmann machine, linear regression, or any combination thereof.

In some cases, the machine learning algorithm may comprise ensemble learning algorithms such as bagging, boosting, and stacking. The machine learning algorithm may be individually applied to the plurality of features. he systems may apply one or more machine learning algorithms.

The predictive model may comprise any number of machine learning algorithms. The random forest machine learning algorithm may be an ensemble of bagged decision trees. The ensemble may be at least about 1, 2, 3, 4, 5, 10, 20, 30, 40, 50, 60, 70, 80, 90, 100, 120, 140, 160, 180, 200, 250, 500, 1000 or more bagged decision trees. The ensemble may be at most about 1000, 500, 250, 200, 180, 160, 140, 120, 100, 90, 80, 70, 60, 50, 40, 30, 20, 10, 5, 4, 3, 2 or less bagged decision trees. The ensemble may be from about 1 to 1000, 1 to 500, 1 to 200, 1 to 100, or 1 to 10 bagged decision trees.

The machine learning algorithms may have a variety of parameters. The variety of parameters may be, for example, learning rate, minibatch size, number of epochs to train for, momentum, learning weight decay, or neural network layers etc.

The learning rate may be between about 0.00001 to 0.1.

The minibatch size may be at between about 16 to 128.

The neural network may comprise neural network layers. The neural network may have at least about 2 to 1000 or more neural network layers.

The number of epochs to train for may be at least about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 150, 200, 250, 500, 1000, 10000, or more.

The momentum may be at least about 0.1, 0.2, 0.3, 0.4, 0.5, 0.6, 0.7, 0.8, 0.9 or more. In some embodiments, the momentum may be at most about 0.9, 0.8, 0.7, 0.6, 0.5, 0.4, 0.3, 0.2, 0.1, or less.

Learning weight decay may be at least about 0.00001, 0.0001, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, or more. In some embodiments, the learning weight decay may be at most about 0.1, 0.09, 0.08, 0.07, 0.06, 0.05, 0.04, 0.03, 0.02, 0.01, 0.009, 0.008, 0.007, 0.006, 0.005, 0.004, 0.003, 0.002, 0.001, 0.0001, 0.00001, or less.

The machine learning algorithm may use a loss function. The loss function may be, for example, regression losses, mean absolute error, mean bias error, hinge loss, Adam optimizer and/or cross entropy.

The parameters of the machine learning algorithm may be adjusted with the aid of a human and/or computer system.

The machine learning algorithm may prioritize certain features. The machine learning algorithm may prioritize features that may be more relevant for detecting cancer. The feature may be more relevant for detecting cancer if the feature is classified more often than another feature in determining cancer. In some cases, the features may be prioritized using a weighting system. In some cases, the features may be prioritized on probability statistics based on the frequency and/or quantity of occurrence of the feature. The machine learning algorithm may prioritize features with the aid of a human and/or computer system.

In some cases, the machine learning algorithm may prioritize certain features to reduce calculation costs, save processing power, save processing time, increase reliability, or decrease random access memory usage, etc.

The computer system **701** may comprise a central processing unit (CPU, also "processor" and "computer processor" herein) **705,** which may be a single core or multi core processor, or a plurality of processor for parallel processing. The computer system **701** may further comprise memory or memory locations **704** (e.g., random-access memory, read-only memory, flash memory), electronic storage unit **706** (e.g., hard disk), communications interface **708** (e.g., network adapter) for communicating with one or more other devices, and peripheral devices **707,** such as cache, other memory, data storage and/or electronic display adapters. The memory **704,** storage unit **706,** interface **708,** and peripheral devices **707** are in communication with the CPU **705** through a communication bus (solid lines), such as a motherboard. The storage unit **706** may be a data storage unit (or a data repository) for storing data, described elsewhere herein. The computer system **701** may be operatively coupled to a computer network ("network") **700** with the aid of the communication interface **708.** The network **700** may be the Internet, intranet, and/or extranet that is in communication with the Internet. The network **700** may, in some case, be a telecommunication and/or data network. The network **700** may include one or more computer servers, which may enable distributed computing, such as cloud computing. The network **700,** in some cases with the aid of the computer system **701,** may implement a peer-to-peer network, which may enable devices coupled to the computer system **701** to behave as a client or a server.

The CPU **705** may execute a sequence of machine-readable instructions, which may be embodied in a program or software. The instructions may be directed to the CPU **705,** which may subsequently program or otherwise configure the CPU **705** to implement methods of the present disclosure, described elsewhere herein. Examples of operations performed by the CPU 705 may include fetch, decode, execute, and writeback.

The CPU **705** may be part of a circuit, such as an integrated circuit. One or more other components of the system **701** may be included in the circuit. In some cases, the circuit is an application specific integrated circuit (ASIC).

The storage unit **706** may store files, such as drivers, libraries, and saved programs. The storage unit **706** may, in addition and/or alternatively, store one or more sequencing reads of one or more subjects' biological sample, downstream sequencing read processes data (e.g., k-mer sequences, cancer mutation abundance, etc.), cancer type (e.g., cancer stage, cancer organ of origin, etc.) if present, treatment administered to treat the cancer, treatment efficacy of the treatment administered, or any combination thereof. The computer system **701,** in some cases may include one or more additional data storage units that are external to the computer system **701,** such as located on a remote server that is in communication with the computer system **701** through an intranet or the internet.

Methods as described herein may be implemented by way of machine (e.g., computer processor) executable code stored on an electronic storage location of the computer device **701,** such as, for example, on the memory **704** or electronic storage unit **706.** The machine executable or machine-readable code may be provided in the form of software. During use, the code may be executed by the processor **705.** In some instances, the code may be retrieved from the storage unit **706** and stored on the memory **704** for ready access by the processor **705.** In some instances, the electronic storage unit **706** may be precluded, and machine-executable instructions are stored on memory **704.**

The code may be pre-compiled and configured for use with a machine having a processor adapted to execute the code or may be compiled during runtime. The code may be supplied in a programming language that may be selected to enable the code to be executed in a pre-complied or as-compiled fashion.

Aspects of the systems and methods provided herein, such as the computer system **701,** may be embodied in programming. Various aspects of the technology may be thought of a "product" or "articles of manufacture" typically in the form of a machine (or processor) executable code and/or associated data that is carried on or embodied in a type of machine readable medium. Machine-executable code may be stored on an electronic storage unit, such memory (e.g., read-only memory, random-access memory, flash memory) or a hard disk. "Storage" type media may include any or all of the tangible memory of a computer, processor the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and/or electromagnetic waves, such as used across physical interfaces between local devices, through wired and optical landline networks and over various air-links. The physical elements that carry such waves, such as wired or wireless links, optical links, or the like, also may be considered as media bearing the software. As used herein, unless restricted to non-transitory, tangible "storage' media, term such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media may include, for example, optical or magnetic disks, such as any of the storage devices in any computer(s) or the like, such as may be used to implement the databases, etc. Volatile storage media include dynamic memory, such as main memory of such a computer platform. Tangible transmission media includes coaxial cables; copper wire and fiber optics, including the wires that comprise a bus within a computer device. Carrier-wave transmission media may take the form of electric or electromagnetic signals, or acoustic or light waves such as those generated during radio frequency (RF) and infrared (IR) data communications. Common forms of computer-readable media therefor include for example: a floppy disk, a flexible disk, hard disk, magnetic tape, any other magnetic medium, a CD-ROM, DVD or DVD-ROM, any other optical medium, punch cards paper tape, any other physical storage medium with pattern of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one more instruction to a processor for execution.

The computer system may include or be in communication with an electronic display **702** that comprises a user interface (UI) **703** for viewing the abundance and prevalence of one or more subjects' k-mer sequences, cancer mutations, suggested therapeutic treatment outputted by a trained predictive model and/or recommendation or determination of a presence or lack thereof cancer for one or more subjects. Examples of UI's include, without limitation, a graphical user interface (GUI) and web-based user interface.

Methods and systems of the present disclosure can be implemented by way of one or more algorithms and with instructions provided with one or more processors as disclosed herein. An algorithm can be implemented by way of software upon execution by the central processing unit 705. The algorithm can be, for example, a machine learning algorithm e.g., random forest, supper vector machines, neural network, and/or graphical models.

In some cases, the disclosure provided herein describes a computer-implemented method for utilizing a trained predictive model to determine the presence or lack thereof cancer of one or more subjects. In some cases, the method may comprise: (a) receiving a plurality of somatic mutations and non-human k-mer sequences of a first one or more subjects' nucleic acid samples; (b) providing as an input to a trained predictive model the first one or more subjects' plurality of somatic mutations and non-human k-mer sequences, wherein the trained predictive model is trained with a second one or more subjects' plurality of somatic mutation sequences, non-human k-mer sequences, and corresponding clinical classifications of the second one or more subjects', and wherein the first one or more subjects and the second one or more subjects are different subjects; and (c) determining the presence or lack thereof cancer of the first one or more subjects based at least in part on an output of the trained predictive model.

In some cases, receiving the plurality of somatic mutations may further comprise counting somatic mutations of the first one or more subjects' nucleic acid samples. In some instances, receiving the plurality of non-human k-mer sequences may comprises counting the non-human k-mer sequences of the first one or more subjects' nucleic acid samples. In some cases, determining the presence or lack thereof cancer of the first one or more subjects may further comprise determining a category or location of the first one or more subjects' cancers. In some instances, determining the presence or lack thereof cancer of the first one or more subjects may further comprise determining one or more types of the first one or more subjects' cancers. In some cases, determining the presence or lack thereof cancer of the first one or more subjects may further comprise determining one or more subtypes of the first one or more subjects' cancers. In some instances, determining the presence or lack thereof cancer of the first one or more subjects may further comprise determining the stage of the cancer, cancer prognosis, or any combination thereof. In some cases, determining the presence or lack thereof cancer of the first one or more subjects may further comprise determining a type of cancer at a low stage. In some instances, the type of cancer at the low-stage may comprise stage I, or stage II cancers. In some cases, determining the presence or lack thereof cancer of the first one or more subjects may further comprise determining the mutation status of the first one or more subjects' cancers. In some cases, the mutation status may comprise malignant, benign, or carcinoma in situ. In some instances, determining the presence or lack thereof cancer of the first one or more subjects may further comprise determining the first one or more subjects' response to a therapy to treat the first one or more subjects' cancers.

In some cases, the cancer determined by the method may comprise: acute myeloid leukemia, adrenocortical carcinoma, bladder urothelial carcinoma, brain lower grade glioma, breast invasive carcinoma, cervical squamous cell carcinoma and endocervical adenocarcinoma, cholangiocarcinoma, colon adenocarcinoma, esophageal carcinoma, glioblastoma multiforme, head and neck squamous cell carcinoma, kidney chromophobe, kidney renal clear cell carcinoma, kidney renal papillary cell carcinoma, liver hepatocellular carcinoma, lung adenocarcinoma, lung squamous cell carcinoma, lymphoid neoplasm diffuse large B-cell lymphoma, mesothelioma, ovarian serous cystadenocarcinoma, pancreatic adenocarcinoma, pheochromocytoma and paraganglioma, prostate adenocarcinoma, rectum adenocarcinoma, sarcoma, skin cutaneous melanoma, stomach adenocarcinoma, testicular germ cell tumors, thymoma, thyroid carcinoma, uterine carcinosarcoma, uterine corpus endometrial carcinoma, uveal melanoma, or any combination thereof.

In some cases, the first one or more subjects and the second one or more subjects may be non-human mammal subjects. In some instances, the first one or more subjects and the second one or more subjects may be human. In some cases, the first one or more subjects and the second one or more subjects may be mammal. In some instances, the plurality of non-human k-mer sequences may originate from the following non-mammalian domains of life: viral, bacterial, archaeal, fungal, or any combination thereof.

Some of the above steps may comprise sub-steps. Many of the steps may be repeated as often as beneficial. One or more of the steps of each of the methods or sets of operations may be performed with circuitry as described herein, for example, one or more of the processor or logic circuitry such as programmable array logic for a field programmable gate array. The circuitry may be programmed to provide one or more of the steps of each of the methods or sets of operations and the program may comprise program instructions stored on a computer readable memory or programmed steps of the logic circuitry such as the programmable array logic or the field programmable gate array, for example.

Additional exemplary embodiments will be further described with reference to the following examples; however, these exemplary embodiments are not limited to such examples.

### EXAMPLES

### Example 1: Training a predictive model to differentiate early-stage lung cancer and lung granulomas

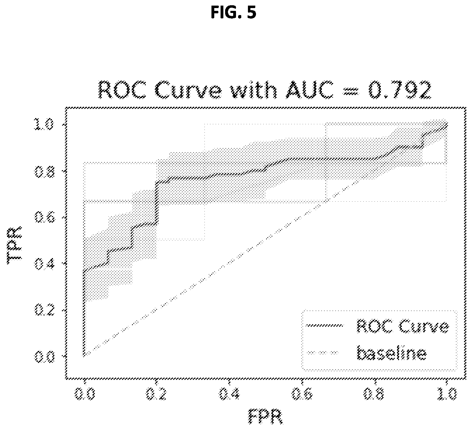

A predictive model was trained with 18 early-stage lung cancer (3 stage II and 15 stage III) and 11 lung granuloma patients' non-mapped cell-free DNA (cfDNA) k-mers and utilized to predict the classification of a patient as having early-stage cancer or lung disease based on their non-mapped cell-free DNA k-mers. Early-stage lung cancer and lung disease patients' cfDNA sequencing reads were mapped to a human genome reference library to separate the mappable human from the unmappable human and non-human sequencing reads. Next, duplicate sequencing reads resulting as an artifact of polymerase chain reaction (PCR) were removed. Gerbil software package was used to extract the prevalence and abundance of all k-mers with a k value of 31 from the unmapped sequencing reads. The k-mer prevalence and abundance was then filtered by removing k-mers identified in blank control samples and k-mer sequences of "GGAAT" and "CCATT" repeat sequences. Next, k-mers with low abundance and low prevalence were filtered. K-mers with abundances of less than 5 instances per sample and prevalence in less than 25 samples of all total samples were removed from the prior filtered k-mer set. A random forest predictive model was then trained with the resulting filtered k-mers and the clinical classification of the patients (i.e., lung cancer or lung disease) with 10-fold cross-validation in a 70:30 training-test data split. The resulting trained predictive model's accuracy was analyzed using receiver operating character area under curve (AUC), as seen in **FIG. 5****,** showing an AUC of 0.792.

### Example 2: Training a predictive model to differentiate stage I lung cancer and lung disease

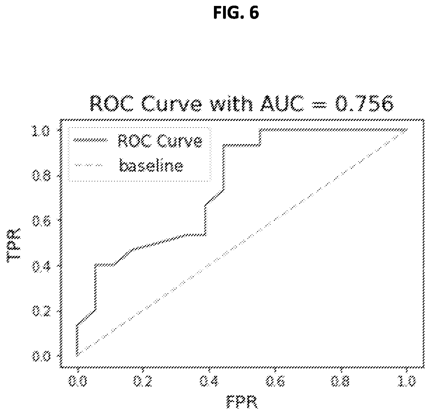

A predictive model was trained with 51 stage I adenocarcinoma lung cancer and 60 lung disease (7 pneumonia, 20 hamartoma, 12 interstitial fibrosis, 5 bronchiectasis, and 16 granulomas) patients' non-mapped cell-free DNA (cfDNA) k-mers and utilized to predict the classification of a patient as having stage I adenocarcinoma or lung disease based on their non-mapped cell-free DNA k-mers. Early-stage lung cancer and lung disease patients' cfDNA sequencing reads were mapped to a human genome reference library to separate the mappable human from the unmappable human and non-human sequencing reads . Next, duplicate sequencing reads resulting as an artifact of polymerase chain reaction (PCR) were removed. Gerbil software package was used to extract the prevalence and abundance of all k-mers with a k value of 31 from the unmapped sequencing reads. The k-mer prevalence and abundance was then filtered by removing k-mers identified in blank control samples and k-mer sequences of "GGAAT" and "CCATT" repeat sequences. Next, k-mers with low abundance and low prevalence were filtered. K-mers with abundances of less than 5 instances per sample and prevalence in less than 20 samples of all total samples were removed from the prior filtered k-mer set. A random forest predictive model was then trained with the resulting filtered k-mers and the clinical classification of the patients (i.e., lung cancer or lung disease) with 10-fold cross-validation in a 70:30 training-test data split. The resulting trained predictive model's accuracy was analyzed using receiver operating character area under curve (AUC), as seen in **FIG. 6****,** showing an AUC of 0.756.

### Example 3: Training a predictive model to classify subjects with an unknown diagnosis of cancer

A predictive model will be trained with known healthy and cancer patients' cell-free DNA to generate a trained predictive model configured to classify an individual suspected of having cancer as healthy or as having cancer. Confirmed healthy and cancer patients' cell-free DNA (cfDNA) will be extracted from a biological samples, e.g., sputum, blood, saliva, or any other bodily fluid with cfDNA, and sequenced. The resulting cfDNA sequencing reads will then be mapped to a human genome library such that exact matching human sequencing reads may be removed from the cfDNA sequencing reads. Next the prevalence and abundance of all k-mers will be extracted from the unmapped sequencing reads. The k-mer sequences will then be filtered for duplicate k-mer sequences that may arise due to the amplification and/or duplication of the cfDNA during library preparation PCR steps. Additionally, k-mers identified in blank control samples and k-mer sequences of "GGAAT" or "CCATT" repeat sequences will be removed. The predictive model will then be trained with the k-mers and corresponding classification (e.g., healthy, or cancerous) of the patients they originated from. The corresponding classification of individuals confirmed to have cancer will include the cancer sub-type, stage, and/or the tissue of origin of the cancer.

A patient suspected of having cancer will then provide a biological sample comprising cfDNA and a similar work flow to the processing of the cfDNA as provided above will be completed. The resulting k-mers will then be provided as an input into the trained predictive model described above. The trained predictive model will then provide a probability of the likelihood that the patient does or does not have cancer. Additionally the trained predictive model will provide the clinical sub-type, stage, and/or the tissue of origin of the cancer identified.

### Example 4: Training a predictive model with a combination of taxonomically assignable and unassignable 'dark matter' reads to classify subjects with an unknown diagnosis of cancer

A predictive model will be trained with known healthy and cancerous patients' cell-free DNA to generate a trained predictive model configured to classify a patient suspected of having cancer as healthy or as having cancer. Confirmed healthy cancer patients' cell-free DNA (cfDNA) will be extracted from a biological sample, e.g., sputum, blood, saliva, or any other bodily fluid with cfDNA, amplified via polymerase chain reaction (PCR), and sequenced. The resulting sequenced cfDNA sequencing reads will then be mapped to a human genome library using exact matching to obtain an output of all unmapped human reads harboring mutations (relative to the selected reference genome build) and all non-human reads. The resulting non-human reads will be taxonomically assigned by alignment to microbial reference genomes via Kraken or bowtie 2 or their equivalents to produce an output of taxonomically assigned microbial reads and their associated abundances. All remaining unmapped non-human reads (comprising, colloquially, sequencing 'dark matter') will be used for k-mer generation. The prevalence and abundance of all dark matter k-mers will be extracted from the dark matter sequencing reads and the prevalence and abundance of all human somatic mutation k-mers will be extracted from the human sequencing reads filtered via strict exact matching to the human reference genome. Next, k-mers identified in blank control samples and k-mer sequences of "GGAAT" or "CCATT" repeat sequences will be removed from the dark matter k-mers. The predictive model will then be trained with a combined dataset comprising the abundances of the human somatic mutation k-mers, the taxonomically assigned microbial reads, and the dark matter k-mers, and corresponding classification (e.g., healthy, or cancerous) of the patients they originated from. The corresponding classification of individuals confirmed to have cancer will include the cancer sub-type, stage, and/or the tissue of origin of the cancer.

A patient suspected of having cancer will then provide a biological sample comprising cfDNA and a similar workflow to the processing of the cfDNA as provided above will be completed to extract human somatic mutations, taxonomically assignable microbes, and dark matter k-mers. The resulting feature set will then be provided as an input into the trained predictive model described above. The trained predictive model will then provide a probability of the likelihood that the patient does or does not have cancer. Additionally the trained predictive model will provide the clinical sub-type, stage, and/or the tissue of origin of the cancer identified.

### Example 5: Training a predictive model with taxonomically assignable k-mers and cancer mutation abundance to classify subjects with an unknown diagnosis of cancer

A predictive model will be trained with known healthy and cancer patients' cell-free DNA to generate a trained predictive model configured to classify an individual suspected of having cancer as healthy or as having cancer, as shown in **FIGS. 1A-1C****.** Confirmed healthy and cancer patients' cell-free DNA (cfDNA) will be extracted from biological samples, e.g., sputum, blood, saliva, or any other bodily fluid with cfDNA, and sequenced. The resulting cfDNA sequencing reads will then be mapped to a human genome library using software package Kraken, such that exact matching human sequencing reads may be removed from the cfDNA sequencing reads leaving non-matching human sequencing reads (i.e., mutated human sequences) and non-human sequencing reads for further analysis. Next software package Bowtie 2 will be used to map the remaining sequencing reads to non-human sequencing reads and mutated human sequencing reads. The mutated human sequencing reads will then be queried against a cancer mutation database to generate a dataset of cancer mutation ID and associated abundance. Next and k-mers will be extracted from the non-human mapped sequencing reads. The k-mer sequences will then be filtered for duplicate k-mer sequences that may arise due to the amplification and/or duplication of the cfDNA during library preparation PCR steps. Additionally, k-mers identified in blank control samples and k-mer sequences of "GGAAT" or "CCATT" repeat sequences will be removed. The predictive model will then be trained with the k-mers, cancer mutation ID and associated abundance, and corresponding classification (e.g., healthy, or cancerous) of the patients they originated from. The corresponding classification of individuals confirmed to have cancer will include the cancer sub-type, stage, and/or the tissue of origin of the cancer.

A patient suspected of having cancer will then provide a biological sample comprising cfDNA and a similar work flow to the processing of the cfDNA as provided above will be completed. The resulting k-mers and cancer mutation ID and abundance will then be provided as an input into the trained predictive model described above. The trained predictive model will then provide a probability of the likelihood that the patient does or does not have cancer. Additionally the trained predictive model will provide the clinical sub-type, stage, and/or the tissue of origin of the cancer identified.

### DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used in the specification and claims, the singular forms "a", "an" and "the" include plural references unless the context clearly dictates otherwise. For example, the term "a sample" includes a plurality of samples, including mixtures thereof.

The terms "determining," "measuring," "evaluating," "assessing," "assaying," and "analyzing" are often used interchangeably herein to refer to forms of measurement. The terms include determining if an element is present or not (for example, detection). These terms can include quantitative, qualitative, or quantitative and qualitative determinations. Assessing can be relative or absolute. "Detecting the presence of" can include determining the amount of something present in addition to determining whether it is present or absent depending on the context.

The terms "subject," "individual," or "patient" are often used interchangeably herein. A "subject" can be a biological entity containing expressed genetic materials. The biological entity can be a plant, animal, or microorganism, including, for example, bacteria, viruses, fungi, and protozoa. The subject can be tissues, cells and their progeny of a biological entity obtained in vivo or cultured in vitro. The subject can be a mammal. The mammal can be a human. The subject may be diagnosed or suspected of being at high risk for a disease. In some cases, the subject is not necessarily diagnosed or suspected of being at high risk for the disease.

The term 'k-mer' is used to describe a specific n-tuple or n-gram of nucleic acid or amino acid sequences that can be used to identify certain regions within biomolecules like DNA. In this embodiment, a k-mer is a short DNA sequence of length "n" typically ranging from 20-100 base pairs derived from metagenomic sequence data.

The terms 'dark matter', 'microbial dark matter', 'dark matter sequencing reads', and 'microbial dark matter sequencing reads' are used to describe non-human sequencing reads that cannot be mapped to known microbial reference genomes and therefore represent nucleic acid sequences that cannot be taxonomically assigned.

The term "in vivo" is used to describe an event that takes place in a subject's body.

The term "ex vivo" is used to describe an event that takes place outside of a subject's body. An ex vivo assay is not performed on a subject. Rather, it is performed upon a sample separate from a subject. An example of an ex vivo assay performed on a sample is an "in vitro" assay.

The term "in vitro" is used to describe an event that takes places contained in a container for holding laboratory reagent such that it is separated from the biological source from which the material is obtained. In vitro assays can encompass cell-based assays in which living or dead cells are employed. In vitro assays can also encompass a cell-free assay in which no intact cells are employed.

As used herein, the term "about" a number refers to that number plus or minus 10% of that number. The term "about" a range refers to that range minus 10% of its lowest value and plus 10% of its greatest value.

Use of absolute or sequential terms, for example, "will," "will not," "shall," "shall not," "must," "must not," "first," "initially," "next," "subsequently," "before," "after," "lastly," and "finally," are not meant to limit scope of the present embodiments disclosed herein but as exemplary.

Any systems, methods, software, compositions, and platforms described herein are modular and not limited to sequential steps. Accordingly, terms such as "first" and "second" do not necessarily imply priority, order of importance, or order of acts.

As used herein, the terms "treatment" or "treating" are used in reference to a pharmaceutical or other intervention regimen for obtaining beneficial or desired results in the recipient. Beneficial or desired results include but are not limited to a therapeutic benefit and/or a prophylactic benefit. A therapeutic benefit may refer to eradication or amelioration of symptoms or of an underlying disorder being treated. Also, a therapeutic benefit can be achieved with the eradication or amelioration of one or more of the physiological symptoms associated with the underlying disorder such that an improvement is observed in the subject, notwithstanding that the subject may still be afflicted with the underlying disorder. A prophylactic effect includes delaying, preventing, or eliminating the appearance of a disease or condition, delaying, or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

The section headings used herein are for organizational purposes only and are not to be construed as limiting the subject matter described.

## Claims

1. A method of diagnosing cancer of a subject, comprising:
(a) determining a plurality of somatic mutations and non-human k-mer sequences of a subject's sample;
(b) comparing the plurality of somatic mutations and the plurality of non-human k-mer sequences of the subject with a plurality of somatic mutations and non-human k-mer sequences for a given cancer; and
(c) diagnosing cancer of the subject by providing a probability of the presence or lack of cancer based at least in part on the comparison of the subject's plurality of somatic mutations and non-human k-mer sequences and the plurality of somatic mutations and non-human k-mer sequences for the given cancer;
wherein step (a) comprises sequencing nucleic acid compositions of a biological sample to generate sequencing reads, isolating sequencing reads to isolate a plurality of filtered sequencing reads, generating the plurality of non-human k-mers from the plurality of filtered sequencing reads, and determining a taxonomy independent abundance of the non-human k-mers; and
wherein step (b) comprises creating a diagnostic model by training a machine learning algorithm with the taxonomy independent abundance of the non-human k-mers.

2. The method of claim 1, wherein determining the plurality of somatic mutations further comprises counting somatic mutations of the subject's sample.

3. The method of claim 1, wherein determining the plurality non-human k-mer sequences comprises counting the non-human k-mer sequences of the subject's sample.

4. The method of claim 1, wherein diagnosing the cancer of the subject further comprises determining a category or location of the cancer.

5. The method of claim 1, wherein diagnosing the cancer of the subject further comprises determining one or more types of the subject's cancer.

6. The method of claim 1, wherein diagnosing the cancer of the subject further comprises determining one or more subtypes of the subject's cancer.

7. The method of claim 1, wherein diagnosing the cancer of the subject further comprises determining the stage of the subject's cancer, cancer prognosis, or any combination thereof.

8. The method of claim 1, wherein diagnosing the cancer of the subject further comprises determining a type of cancer at a low-stage.

9. The method of claim 8, wherein the type of cancer at the low-stage comprises stage I, or stage II cancers.

10. The method of claim 1, wherein diagnosing the cancer of the subject further comprises determining the mutation status of the subject's cancer.

11. The method of claim 1, wherein diagnosing the cancer of the subject further comprises determining the subject's response to therapy to treat the subject's cancer.

12. The method of claim 1, wherein the cancer comprises: lung adenocarcinoma, lung squamous cell carcinoma, , or any combination thereof.

13. The method of claim 1, wherein the subject is a human.

14. The method of claim 1, where the subject is a mammal.

15. The method of claim 1, wherein the plurality of non-human k-mer sequences originate from the following non-mammalian domains of life: viral, bacterial, archaeal, fungal, or any combination thereof.

## Patentansprüche

1. Verfahren zur Diagnosestellung von Krebs bei einem Individuum, umfassend:
(a) Bestimmen einer Mehrzahl somatischer Mutationen und nicht-humane k-mer-Sequenzen einer Probe des Individuums;
(b) Vergleichen der Mehrzahl somatischer Mutationen und der Mehrzahl nicht-humane k-mer-Sequenzen des Individuums mit einer Mehrzahl somatischer Mutationen und nicht-humane k-mer-Sequenzen für einen gegebenen Krebs; und
(c) Diagnosestellung von Krebs bei dem Individuum durch Angabe einer Wahrscheinlichkeit für das Vorliegen oder das Fehlen eines Krebses basierend wenigstens teilweise auf dem Vergleich der Mehrzahl somatischer Mutationen und nicht-humane k-mer-Sequenzen des Individuums und der Mehrzahl somatischer Mutationen und nicht-humane k-mer-Sequenzen für den gegebenen Krebs;
wobei Schritt (a) Folgendes umfasst: Sequenzieren von Nukleinsäurezusammensetzungen einer biologischen Probe zum Erzeugen von Sequenzierungsreads, Isolieren von Sequenzierungsreads zum Isolieren einer Mehrzahl gefilterter Sequenzierungsreads, Erzeugen der Mehrzahl nicht-humane k-mers aus der Mehrzahl gefilterter Sequenzierungsreads und Bestimmen einer taxonomieunabhängigen Häufigkeit der nicht-humane k-mers; und
wobei Schritt (b) Folgendes umfasst: Erstellen eines diagnostischen Modells durch Trainieren eines maschinellen Lernalgorithmus mit der taxonomieunabhängigen Häufigkeit der nicht-humane k-mers.

2. Verfahren von Anspruch 1, wobei das Bestimmen der Mehrzahl somatischer Mutationen ferner Zählen somatischer Mutationen der Probe des Individuums umfasst.

3. Verfahren von Anspruch 1, wobei das Bestimmen der Mehrzahl nicht-humane k-mer-Sequenzen Zählen der nicht-humane k-mer-Sequenzen der Probe des Individuums umfasst.

4. Verfahren von Anspruch 1, wobei die Diagnosestellung des Krebses des Individuums ferner Bestimmen einer Kategorie oder eines Ortes des Krebses umfasst.

5. Verfahren von Anspruch 1, wobei die Diagnosestellung des Krebses des Individuums ferner Bestimmen eines oder mehrerer Typen des Krebses des Individuums umfasst.

6. Verfahren von Anspruch 1, wobei die Diagnosestellung des Krebses des Individuums ferner Bestimmen eines oder mehrerer Subtypen des Krebses des Individuums umfasst.

7. Verfahren von Anspruch 1, wobei die Diagnosestellung des Krebses des Individuums ferner Bestimmen des Stadiums des Krebses des Individuums, einer Krebsprognose oder einer beliebigen Kombination davon umfasst.

8. Verfahren von Anspruch 1, wobei die Diagnosestellung des Krebses des Individuums ferner Bestimmen eines Krebs-Typs in einem niedrigen Stadium umfasst.

9. Verfahren von Anspruch 8, wobei der Krebs-Typ in dem niedrigen Stadium Stadium-I- oder Stadium-II-Krebs umfasst.

10. Verfahren von Anspruch 1, wobei die Diagnosestellung des Krebses des Individuums ferner Bestimmen des Mutationsstatus des Krebses des Individuums umfasst.

11. Verfahren von Anspruch 1, wobei die Diagnosestellung des Krebses des Individuums ferner Bestimmen der Reaktion des Individuums auf eine Therapie zur Behandlung des Krebses des Individuums umfasst.

12. Verfahren von Anspruch 1, wobei der Krebs Folgendes umfasst: Lungenadenokarzinom, Lungenplattenepithelkarzinom oder eine beliebige Kombination davon.

13. Verfahren von Anspruch 1, wobei das Individuum ein Mensch ist.

14. Verfahren von Anspruch 1, wobei das Individuum ein Säugetier ist.

15. Verfahren von Anspruch 1, wobei die Mehrzahl nicht-humane k-mer-Sequenzen aus den folgenden nicht-säugetierartigen Lebensdomänen stammt: viral, bakteriell, archaeal, pilzhaft oder eine beliebige Kombination davon.

## Revendications

1. Procédé de diagnostic de cancer d'un sujet, comprenant :
(a) la détermination d'une pluralité de mutations somatiques et de séquences k-mères non humaines d'un l'échantillon du sujet ;
(b) la comparaison de la pluralité de mutations somatiques et de la pluralité de séquences k-mères non humaines du sujet avec une pluralité de mutations somatiques et de séquences k-mères non humaines pour un cancer donné ; et
(c) le diagnostic de cancer du sujet en fournissant une probabilité de la présence ou de l'absence de cancer fondée au moins en partie sur la comparaison de la pluralité de mutations somatiques et de séquences k-mères non humaines du sujet et de la pluralité de mutations somatiques et de séquences k-mères non humaines pour le cancer donné ;
dans lequel l'étape (a) comprend le séquençage de compositions d'acides nucléiques d'un échantillon biologique pour générer des lectures de séquençage, l'isolement des lectures de séquençage pour isoler une pluralité de lectures de séquençage filtrées, la génération de la pluralité de k-mères non humaines à partir de la pluralité de lectures de séquençage filtrées, et la détermination d'une abondance indépendante de la taxonomie des k-mères non humaines ; et
dans lequel l'étape (b) comprend la création d'un modèle de diagnostic par entraînement d'un algorithme d'apprentissage automatique avec l'abondance indépendante de la taxonomie des k-mères non humaines.

2. Procédé selon la revendication 1, dans lequel la détermination de la pluralité de mutations somatiques comprend en outre le comptage des mutations somatiques de l'échantillon du sujet.

3. Procédé selon la revendication 1, dans lequel la détermination de la pluralité de séquences k-mères non humaines comprend le comptage des séquences k-mères non humaines de l'échantillon du sujet.

4. Procédé selon la revendication 1, dans lequel le diagnostic du cancer du sujet comprend en outre la détermination d'une catégorie ou d'un emplacement du cancer.

5. Procédé selon la revendication 1, dans lequel le diagnostic du cancer du sujet comprend en outre la détermination d'un ou plusieurs types du cancer du sujet.

6. Procédé selon la revendication 1, dans lequel le diagnostic du cancer du sujet comprend en outre la détermination d'un ou plusieurs sous-types du cancer du sujet.

7. Procédé selon la revendication 1, dans lequel le diagnostic du cancer du sujet comprend en outre la détermination du stade du cancer du sujet, du pronostic du cancer ou de toute combinaison de ceux-ci.

8. Procédé selon la revendication 1, dans lequel le diagnostic du cancer du sujet comprend en outre la détermination d'un type de cancer à un stade précoce.

9. Procédé selon la revendication 8, dans lequel le type de cancer au stade précoce comprend les cancers de stade I ou de stade II.

10. Procédé selon la revendication 1, dans lequel le diagnostic du cancer du sujet comprend en outre la détermination de l'état de mutation du cancer du sujet.

11. Procédé selon la revendication 1, dans lequel le diagnostic du cancer du sujet comprend en outre la détermination de la réponse du sujet à une thérapie pour traiter le cancer du sujet.

12. Procédé selon la revendication 1, dans lequel le cancer comprend : l'adénocarcinome du poumon, le carcinome épidermoïde du poumon, ou toute combinaison de ceux-ci.

13. Procédé selon la revendication 1, dans lequel le sujet est un humain.

14. Procédé selon la revendication 1, dans lequel le sujet est un mammifère.

15. Procédé selon la revendication 1, dans lequel la pluralité de séquences k-mères non humaines proviennent des domaines de vie non mammifères suivants : viral, bactérien, archéen, fongique ou toute combinaison de ceux-ci.
